# EUROPEAN PATENT SPECIFICATION

(11) **EP 4 412 583 B1**
(45) Date of publication and mention of the grant of the patent: **08.07.2026**
(21) Application number: 22782593.2
(22) Date of filing: 03.10.2022
(51) Int. Cl.: A61K 9/00, A61K 47/08, A61K 47/14, C07D 311/20, C08G 65/00, C08L 71/02

(54) **SURFACTANT STABILIZERS**
TENSIDSTABILISATOREN
STABILISATEURS D'AGENTS TENSIOACTIFS

(30) Priority: 04.10.2021 EP 21200819
(43) Date of publication of application: 14.08.2024
(73) Proprietor: Novartis AG, 4056 Basel (CH)
(72) Inventor: BECHTOLD-PETERS, Karoline, 4002 Basel (CH); BROSIG, Sebastian, 4002 Basel (CH); GALLOU, Fabrice, 4002 Basel (CH); PARMENTIER, Michael, 4002 Basel (CH); SERNO, Tim, 4002 Basel (CH); THAKARE, Vivek Sudam, 6250 Kundl (AT)
(74) Representative: Kuhn, Dieter
(86) International application number: PCT/IB2022/059404
(87) International publication number: WO 2023/057871

(56) References cited:
- WO-A1-2013/135892
- WO-A1-2018/134710
- WO-A1-2020/097141
- US-A1- 2015 110 924
- US-A1- 2017 182 133
- AGUIRRE-RAMÍREZ M ET AL: "Surfactants: physicochemical interactions with biological macromolecules", BIOTECHNOLOGY LETTERS, vol. 43, no. 3, 3 February 2021 (2021-02-03), pages 523 - 535, XP037362888, ISSN: 0141-5492, DOI: 10.1007/S10529-020-03054-1
- ZHIPING ZHANG ET AL: "Vitamin E TPGS as a molecular biomaterial for drug delivery", BIOMATERIALS, ELSEVIER, AMSTERDAM, NL, vol. 33, no. 19, 13 March 2012 (2012-03-13), pages 4889 - 4906, XP028413201, ISSN: 0142-9612, [retrieved on 20120322], DOI: 10.1016/J.BIOMATERIALS.2012.03.046

## Description

### FIELD OF THE INVENTION

The present invention is directed to improved protein-containing formulations, stabilized or inhibited against protein aggregation, comprising an amphiphilic surfactant. The formulation comprises a protein, such as a disordered protein, antibody or enzyme, and an amphiphilic surfactant, in particular, a Vitamin E analog, Sitosterol analog or poly(alkylene glycol) alkyl ether analog as a stabilizer. The use of an amphiphilic surfactant leads to an improved formulation, which prevents or suppresses aggregation of the protein. The invention therefore provides a stabilized formulation comprising a protein and an amphiphilic surfactant, and a method of stabilizing a protein-containing formulation using said surfactant. The invention further provides the use of an amphiphilic surfactant as a stabilizer for protein-containing formulations.

### BACKGROUND

Protein based pharmaceuticals have limited shelf lives and are often structurally and functionally unstable, requiring them to be produced, transported, and stored using a system of refrigerators and freezers known as the "cold-chain." This makes many of these lifesaving drugs difficult and expensive to manufacture and deliver.

Although numerous molecules are used as crowding agents to stabilize pharmaceuticals in liquid formulations, these additives can be flawed. For example, non-reducing sugars like manitol, sorbitol, and trehalose are effective in solution but are prone to crystallization and phase separation upon freezing. (Shire, S. J. Curr. Opin. Biotechnol. 20, 708-714 (2009)). Sucrose does not have this problem, but its hydrolysis results in unwanted glycosylation of pharmaceuticals (Shire, S. J. Curr. Opin. Biotechnol. 20, 708-714 (2009)). Surfactants are also common additives; however, surfactants, such as polysorbate 20 and 80, suffer from various chemical and enzymatic degradation, moreover they produce peroxides that oxidize methionine groups (Shire, S. J. Curr. Opin. Biotechnol. 20, 708-714 (2009)).

US2015110924A1 discloses compositions comprising a modified food starch and one or more non-polar compounds. In some instances, the compositions contain a watersoluble derivative of vitamin E mixture, containing relatively high concentrations of dimer forms of the PEG-derivative of vitamin E. US201715461389A discloses compositions comprising a mucoadhesive protein and a surface active agent selected from among one or more of a PEG-derivative of vitamin E that is a polyalkylene glycol derivative of vitamin E. WO2018134710A1 discloses compositions comprising an enzyme, and a surfactant comprising a vitamin E-derived compound having a D-α-tocopherol poly(ethylene glycol) succinate (TPGS), preferably TPGS-750-M, TPGS-1000, PTS (PEG-600/α-tocopherol-based diester of sebacic acid.

Some protein-based pharmaceuticals can be stored at room temperature if they are lyophilized (freeze dried); however, most protein-based pharmaceuticals denature as a result of either the freezing or drying process. Sometimes surfactants in addition to crowding agents can protect protein-based pharmaceuticals during lyophilization, but none of these crowding agents work universally. The most effective additives for a given pharmaceutical is highly dependent on factors including the isoelectric point, β-sheet content, and melting temperature of the drug (Roughton et al. Comput. Chem. Eng. 58, 369-377 (2013)). Even with the addition of stabilizers, many protein-based pharmaceuticals are too unstable to survive lyophilization (Roughton et al. Comput. Chem. Eng. 58, 369-377 (2013)).

Surfactants are amphiphilic molecules often added to biopharmaceutical formulations in order to stabilize biologics (API) from stress encountered during manufacturing, transport, storage and administration. The exact mode of action depends on the nature of the surfactant and structural properties of the stabilized biopharmaceutical, yet, two main mechanisms have been proposed to explain their protective effect i) displacement mechanism and ii) preferential association.

As a downside many commercially established surfactants, namely polysorbate 20 and polysorbate 80, suffer from various chemical and enzymatic degradation mechanisms such as autoxidation, hydrolysis and enzymatic degradation by esterases and lipases. The resulting insoluble free fatty acids can lead to visible and sub visible particles which are of special concern to regulatory agencies and pose a risk for immunogenicity. Moreover, polysorbates degradation products result in the formation of peroxides when subjected to light, which in turn is a trigger for protein oxidation and surfactant autoxidation. In addition, polysorbate synthesis suffers from production related impurities such as remaining free fatty acids or lot to lot inconsistency, which is another critical factor governing polysorbate stability.

When preparing a pharmaceutical formulation which should be physico-chemical acceptable, and stable for a long time, consideration can not only be taken to the physiological properties of the protein but also other aspects must be considered such as the industrial manufacture, easy handling for the patient and safety for the patient. The results of these aspects are not predictable when testing different formulations and there is often a unique set of solutions for each protein.

Hence, there is a need in the art to provide stable protein-containing formulations suitable for parenteral administration to patients, *e.g.,* for intravenous, intramuscular or subcutaneous administration.

The present invention overcomes previous shortcomings in the art by providing new formulations and methods for stabilizing proteins.

### SUMMARY OF INVENTION

The present invention is based on the findings that a significantly higher stabilization effect is achieved by using an amphiphilic surfactant as a stabilizer in protein-containing formulations as compared to conventional stabilizers for pharmaceutical formulations such as polysorbates.

Surprisingly, amphiphilic surfactants which are not derived from fatty acid building blocks, have been found to generate fewer impurities and display a better surfactant stability profile compared to the currently used polysorbates.

Specifically, stable protein-containing formulations that have much lower levels of protein aggregation can be prepared by adding an amphiphilic surfactant as a stabilizer.

Surprisingly, it was observed that changes in the surfactant linker building block significantly affected the surfactant safety profile in human primary tissue, which to our knowledge is unknown in the field.

Thus, the present invention relates to stable protein-containing formulations that comprise an amphiphilic surfactant and methods for suppressing protein aggregation formation by adding an amphiphilic surfactant (e.g., Vitamin E) as a stabilizer to the protein-containing formulations.

In a first aspect, the present invention provides a stable protein-containing formulation comprising an amphiphilic surfactant, wherein the amphiphilic surfactant is a compound of formula (I): wherein: 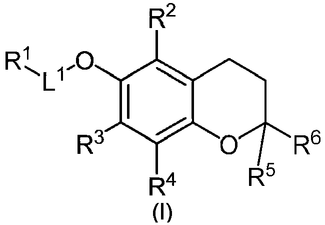
L¹ is -C(=O)-X-C(=O)- ;
X is a branched C₂-C₂₀alkylene; R¹ is a poly(C₁-C₄-alkylene glycol) group, *e.g.,* PEG group, optionally wherein the poly(C₁-C₄-alkylene glycol) group terminates in hydroxyl or C₁-C₆alkoxyl;
R², R³, R⁴ and R⁵ are each independently selected from hydrogen and C₁-C₁₀alkyl; and
R⁶ is C₅-C₂₀alkyl.

The above aspects can be combined. Other objects, features, advantages and aspects of the present invention will become apparent to those skilled in the art from the following description and appended Embodiments. It should be understood, however, that the following description, appended Embodiments, and specific examples, which indicate preferred embodiments of the application, are given by way of illustration only.

### Brief Description of the drawings

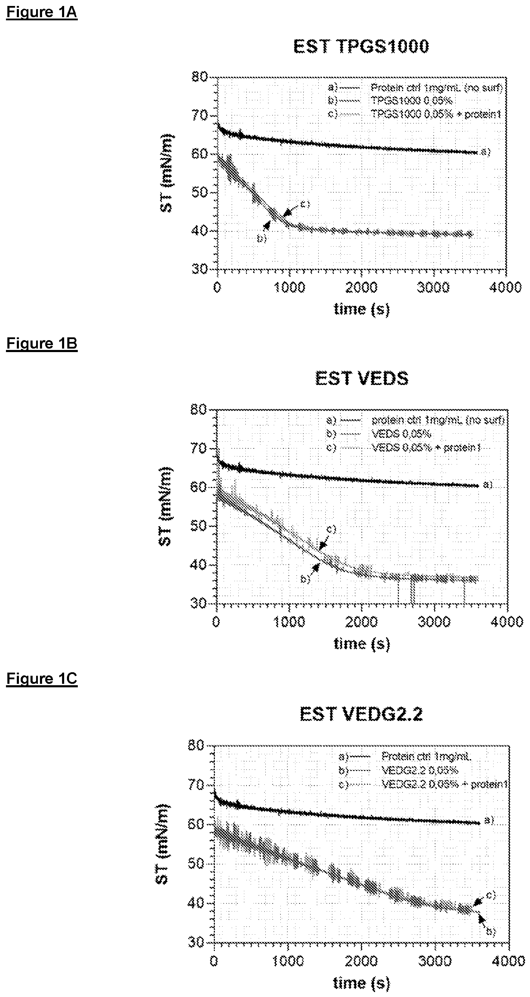

Fig. 1A shows the equilibrium surface tension of a histidine formulation after one hour. Samples measured were a) protein in buffer; b) surfactant (TPGS1000) in buffer and the combination c) protein and surfactant in buffer. The surface active protein lowers the surface tension throughout the experiment whereas the used surfactant exceeds protein surface activity which suggests that the protein is displaced from the interface.
Fig. 1B shows the equilibrium surface tension of a histidine formulation after one hour. Samples measured were a) protein in buffer; b) surfactant Vitamin E dimethyl succinate ((VEDS), Example 10) in buffer and the combination c) protein and surfactant in buffer. The surface active protein lowers the surface tension throughout the experiment whereas the used surfactant exceeds protein surface activity which suggests that the protein is displaced from the interface.
Fig. 1C shows the equilibrium surface tension of a histidine formulation after one hour. Samples measured were a) protein in buffer; b) surfactant Vitamin E dimethyl glutarate (VEDG2.2, Example 12) in buffer and the combination c) protein and surfactant in buffer. The surface active protein lowers the surface tension throughout the experiment whereas the used surfactant exceeds protein surface activity which suggests that the protein is displaced from the interface.

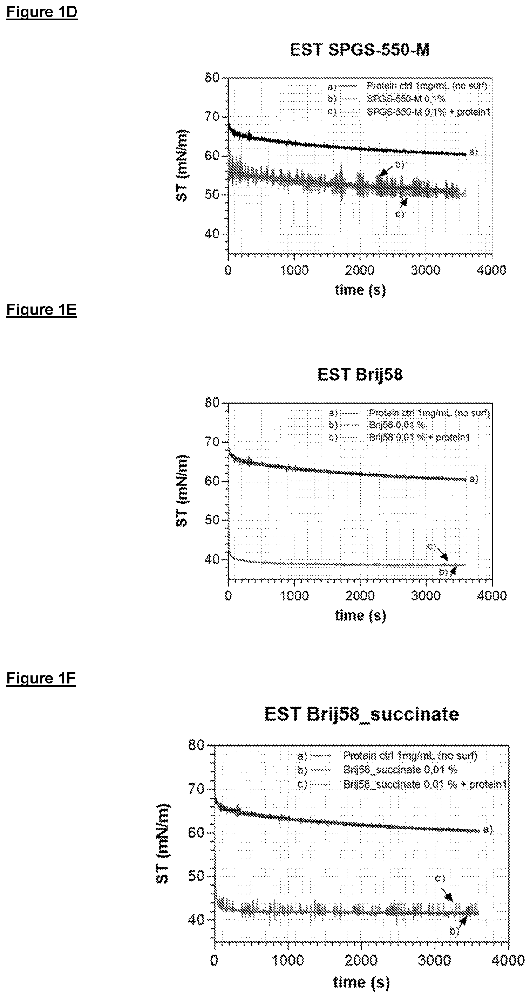

Fig. 1D shows the equilibrium surface tension of a histidine formulation after one hour. Samples measured were a) protein in buffer; b) surfactant (SPGS-550-M) in buffer and the combination c) protein and surfactant in buffer. The surface active protein lowers the surface tension throughout the experiment whereas the used surfactant exceeds protein surface activity which suggests that the protein is displaced from the interface.
Fig. 1E shows the equilibrium surface tension of a histidine formulation after one hour. Samples measured were a) protein in buffer; b) surfactant (Brij58) in buffer and the combination c) protein and surfactant in buffer. The surface active protein lowers the surface tension throughout the experiment whereas the used surfactant exceeds protein surface activity which suggests that the protein is displaced from the interface.
Fig. 1F shows the equilibrium surface tension of a histidine formulation after one hour. Samples measured were a) protein in buffer; b) surfactant (Brij58 succinate, Example 1) in buffer and the combination c) protein and surfactant in buffer. The surface active protein lowers the surface tension throughout the experiment whereas the used surfactant exceeds protein surface activity which suggests that the protein is displaced from the interface.

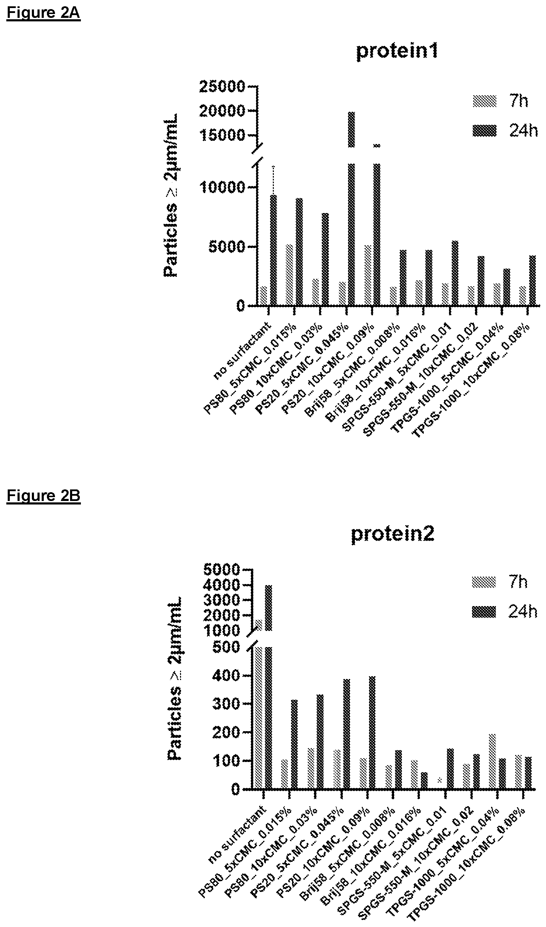

Fig. 2A shows the particle count ≥2 µm/mL, generated through orbital shaking of protein 1 in liquid formulation for 7 h and 24 h. The assay mimics mechanical stress. The data show less particle creation in disordered protein samples containing amphiphilic surfactants and non-fatty acid based surfactants outperforming the current standard polysorbates.
Fig. 2B shows the particle count ≥2 µm/mL, generated through orbital shaking of protein 2 in liquid formulation for 7 h and 24 h. The assay mimics mechanical stress. The data show less particle creation in Fab protein samples containing amphiphilic surfactants and non-fatty acid based surfactants outperforming the current standard polysorbate.

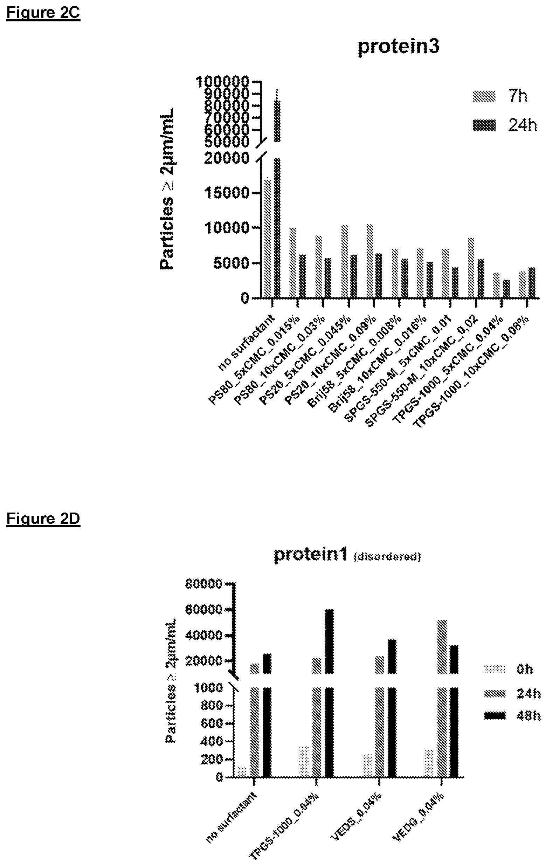

Fig. 2C shows the particle count ≥2 µm/mL, generated through orbital shaking of protein 3 in liquid formulation for 7 h and 24 h. The assay mimics mechanical stress. The data show comparable or less particle creation in mAb protein samples containing amphiphilic surfactants and non-fatty acid based surfactants outcompeting the current standard polysorbate.
Fig. 2D shows the particle count ≥2 µm/mL, generated through orbital shaking of protein 1 in liquid formulation for 0 h, 7 h and 24h. The assay mimics mechanical stress. The data show less particle creation in disordered protein samples containing VEDS or VEDG compared to TPGS-1000.

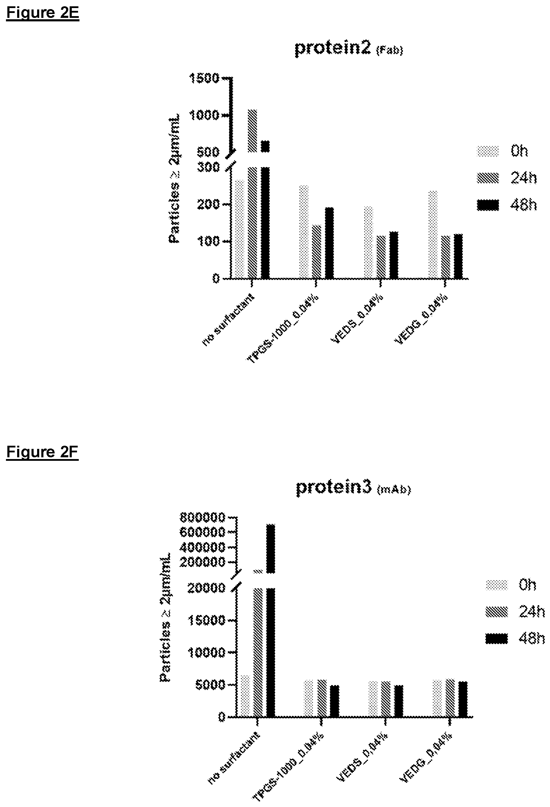

Fig. 2E shows the particle count ≥2 µm/mL, generated through orbital shaking of protein 2 in liquid formulation for 0 h, 7 h and 24 h. The assay mimics mechanical stress. The data show less particle creation in Fab protein samples containing VitaminE based surfactants compared to no surfactant control samples.
Fig. 2F shows the particle count ≥2 µm/mL, generated through orbital shaking of protein 3 in liquid formulation for 0 h, 7 h and 24 h. The assay mimics mechanical stress. The data show less particle creation in mAb protein samples containing VitaminE based surfactants compared to no surfactant control samples.

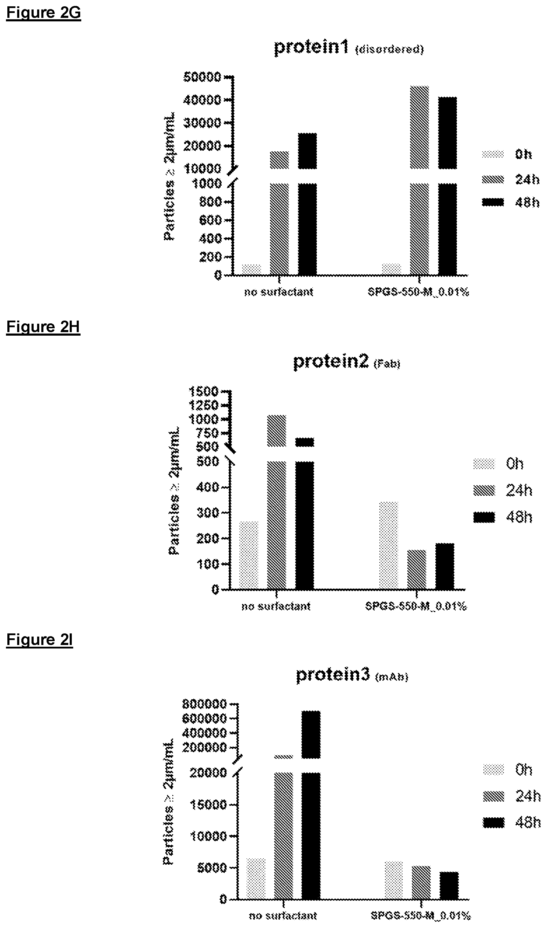

Fig. 2G shows the particle count ≥2 µm/mL, generated through orbital shaking of protein 1 in liquid formulation for 0 h, 7 h and 24 h. The assay mimics mechanical stress. The data show particle creation in disordered protein samples containing beta-sitosterol based surfactants compared to no surfactant control samples.
Fig. 2H shows the particle count ≥2 µm/mL, generated through orbital shaking of protein 2 in liquid formulation for 0 h, 7 h and 24 h. The assay mimics mechanical stress. The data show less particle creation in Fab protein samples containing beta-sitosterol based surfactants compared to no surfactant control samples.
Fig. 2I shows the particle count ≥2 µm/mL, generated through orbital shaking of protein 3 in liquid formulation for 0 h, 7 h and 24 h. The assay mimics mechanical stress. The data show less particle creation in mAb protein samples containing beta-sitosterol based surfactants compared to no surfactant control samples.

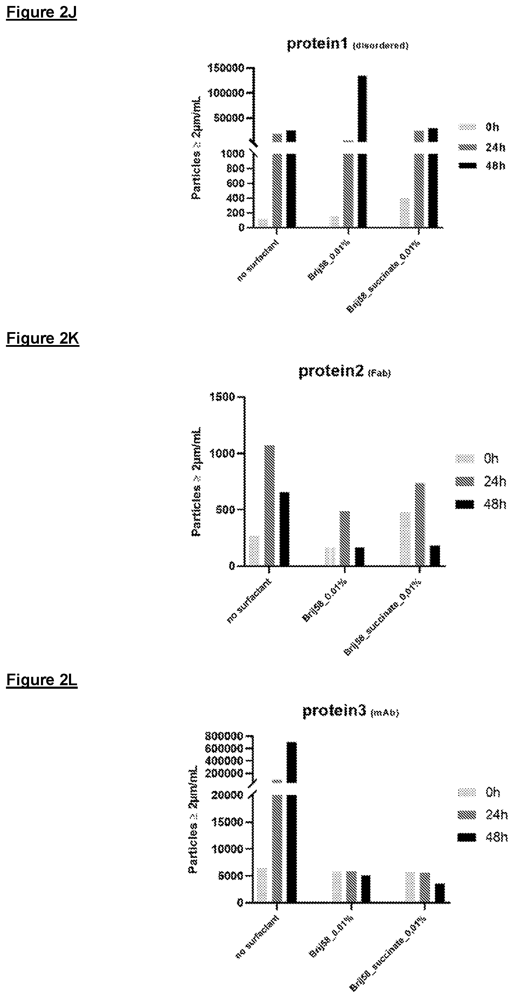

Fig. 2J shows the particle count ≥2 µm/mL, generated through orbital shaking of protein 1 in liquid formulation for 0 h, 7 h and 24 h. The assay mimics mechanical stress. The data show less particle creation in disordered protein samples containing Brij58_succinate compared to protein samples containing Brij58.
Fig. 2K shows the particle count ≥2 µm/mL, generated through orbital shaking of protein 2 in liquid formulation for 0 h, 7 h and 24 h. The assay mimics mechanical stress. The data show less particle creation in Fab protein samples containing Brij58 based surfactants compared to no surfactant control samples.
Fig. 2L shows the particle count ≥2 µm/mL, generated through orbital shaking of protein 3 in liquid formulation for 0 h, 7 h and 24 h. The assay mimics mechanical stress. The data show less particle creation in mAb protein samples containing Brij58 based surfactants compared to no surfactant control samples.

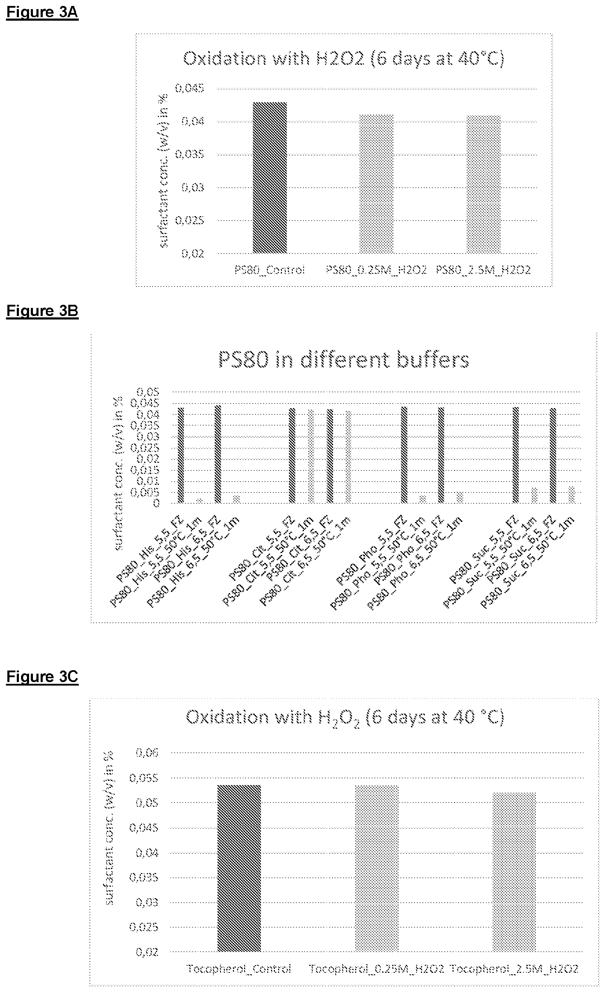

Fig. 3A shows the polysorbate content in % (w/v) after oxidative stress (H₂O₂) for 6 d at 40 °C compared to a non-treated control. The data show minor loss of PS80 upon H₂O₂ treatment.
Fig. 3B shows the polysorbate content in % (w/v) after pH and temperature stress for 1 month at 50 °C compared to a non-treatment control (FZ). The study utilized four representative buffers typically used for biologics formulations. PS80 appears to be stable only in citrate buffer. PS80 is degraded in Histidine, Phosphate and Succinate buffer.
Fig. 3C shows the TPGS1000 content in % (w/v) after oxidative stress (H₂O₂) for 6 d at 40 °C compared to a non-treated control. TPGS1000 is considered stable upon H₂O₂ treatment.

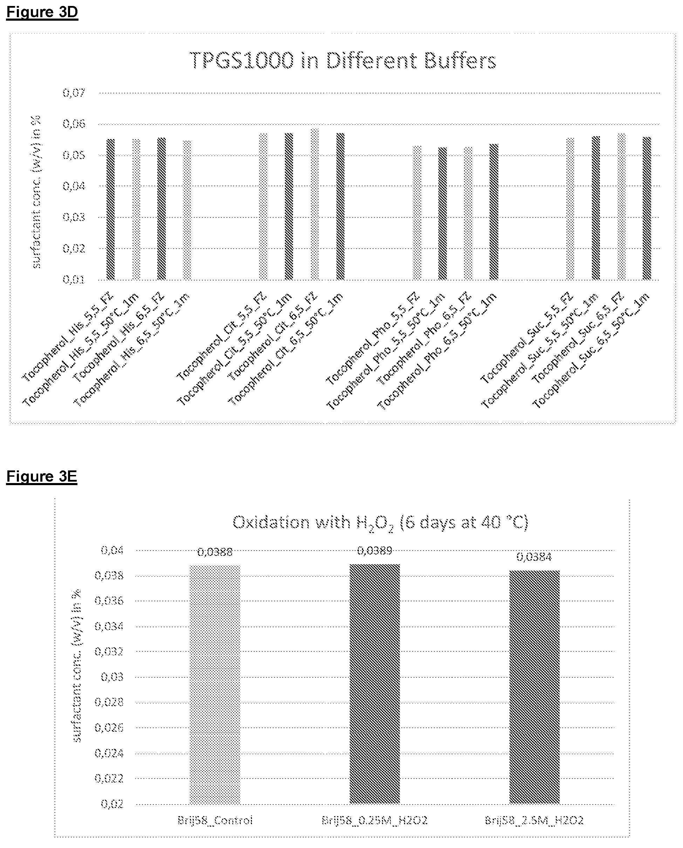

Fig. 3D shows the TPGS1000 content in % (w/v) after pH and temperature stress for 1 month at 50 °C compared to a non-treatment control (FZ). The study utilized four representative buffers used for biologics formulation. TPGS1000 is considered stable in all used buffer conditions.
Fig. 3E shows the Brij58 content in % (w/v) after oxidative stress (H₂O₂) for 6d at 40°C compared to a non-treated control. Brij58 is considered stable upon H₂O₂ treatment.

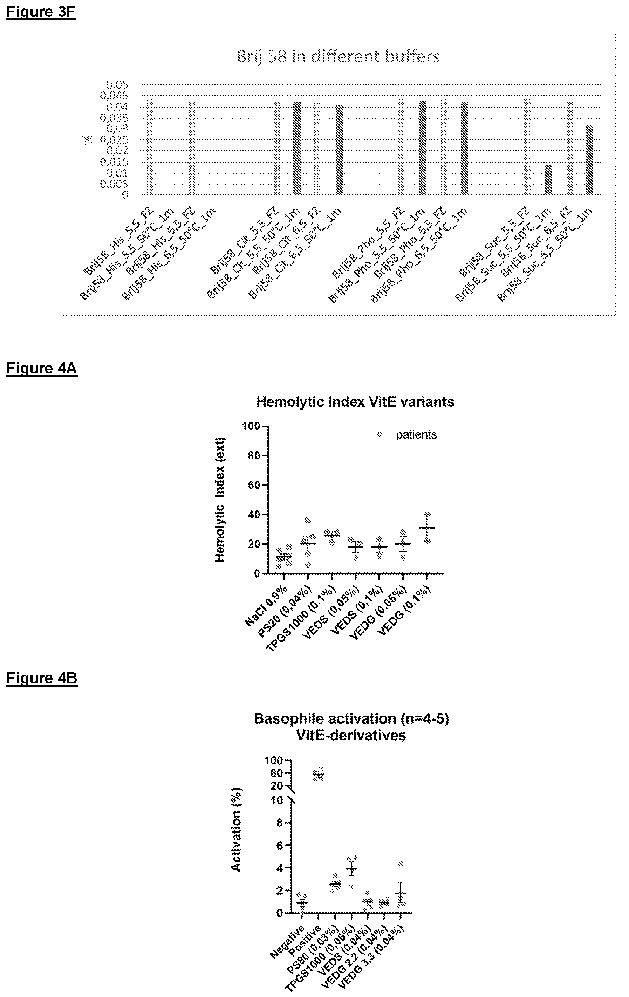

Fig. 3F shows the Brij58 content in % (w/v) after pH and temperature stress for 1 month at 50 °C compared to a non-treatment control (FZ). The study utilized four representative buffers used for biologics formulation. Brij58 is considered stable in Citrate and Phosphate buffer conditions. Brij58 is degraded in Histidine and Succinate buffer conditions.
Fig. 4A shows the hemolytic potential of VitaminE based surfactants compared to a polysorbate and no-surfactant (NaCl) control sample. The safety profile of tested VitaminE based surfactants is comparable or better than the polysorbate reference.
Fig. 4B shows the number of activated B cells (%) upon surfactant treatment. Vitamin E derivatives were compared to polysorbate and a B-Cell activating control (positive). TPGS1000 shows a level of B-cell activation which is comparable to polysorbate. Vitamin E derivatives VEDS, VEDG 2.2 and VEDG 3.3 show a lower level of B-cell activation than TPGS1000.

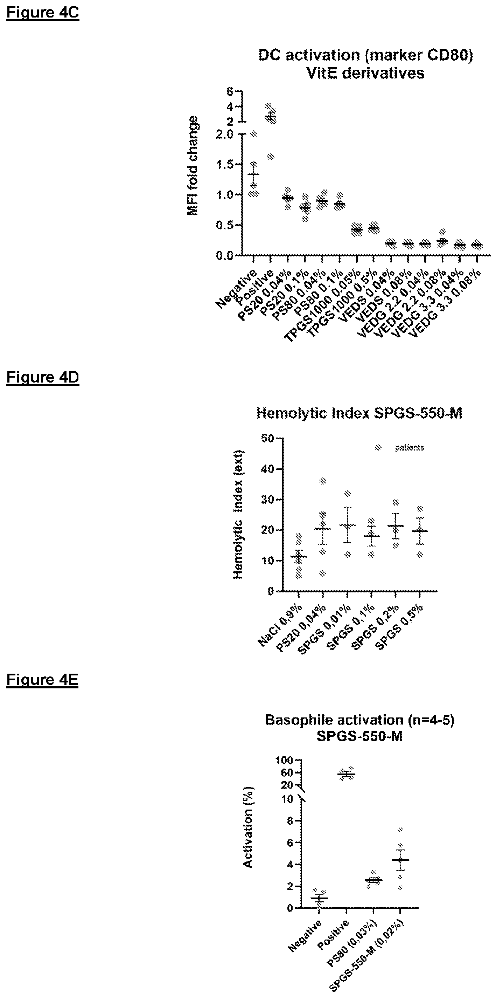

Fig. 4C shows the mean fluorescence intensity (MFI) fold change of LPS activated dendritic cells (DC) compared to no-treatment control and Vitamin E derivatives treated cells. Vitamin E derivatives VEDS, VEDG 2.2 and VEDG 3.3 show no DC activation.
Fig. 4D shows the hemolytic potential of beta sitosterol based surfactants in increasing concentrations compared to a polysorbate and no-surfactant (NaCl) control sample. The safety profile of tested VitaminE based surfactants is comparable to the polysorbate reference.
Fig. 4E shows the number of activated B cells (%) upon surfactant treatment. Beta sitosterol based SPGS-550-M was compared to polysorbate and a B-Cell activating control (positive). Beta sitosterol shows slightly increased levels of B-cell activation which is still regard as safe.

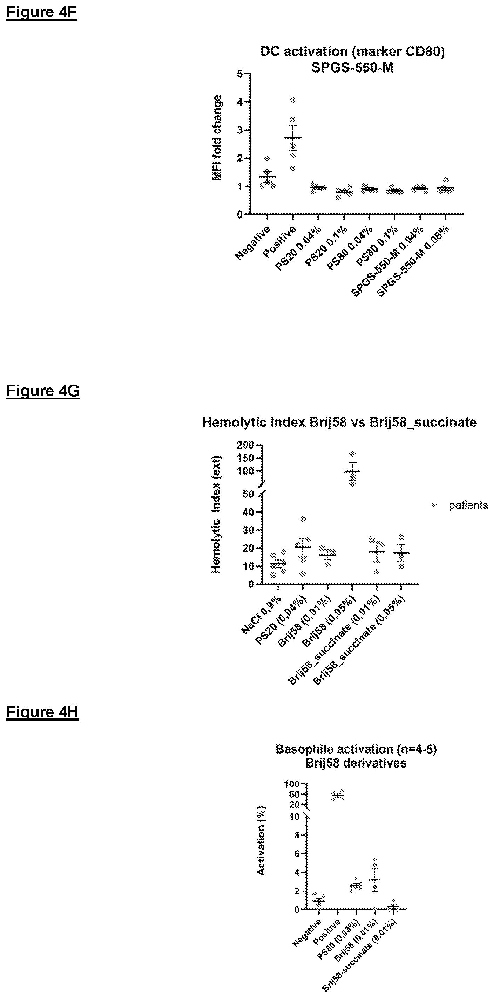

Fig. 4F shows the mean fluorescence intensity (MFI) fold change of LPS activated dendritic cells (DC) compared to the no-treatment control (cell medium) and beta sitosterol based SPGS-550-M treated cells. SPGS-550-M shows no DC activation.
Fig. 4G shows the hemolytic potential of Brij58 based surfactants in increasing concentrations compared to a polysorbate and no-surfactant (NaCl) control sample. Elevated levels of Brij58 (0.05 % w/v) show increased hemolytic potential and can be regarded as not safe. The hemolytic potential was decreased for Brij58succinate which contained a succinate linker in between the lipophilic and hydrophilic portion. The safety profile of tested Brij58succinate is comparable to the polysorbate reference.
Fig. 4H shows the number of activated B cells (%) upon surfactant treatment. Brij58 and Brij58 succinate treated cells were compared to polysorbate and a B-Cell activating control (positive). Brij58 shows slight patient specific B-cell activation which on average of all patients is regarded safe. Brij58 succinate shows no B-cell activation.

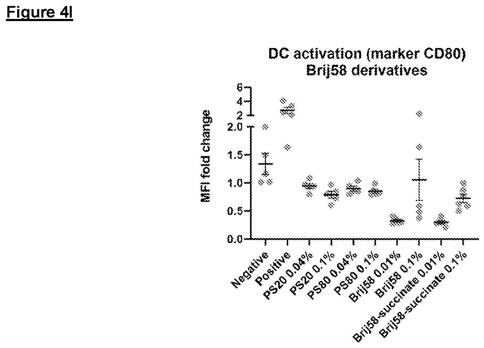

Fig. 4I shows the mean fluorescence intensity (MFI) fold change of LPS activated dendritic cells (DC) compared to the no-treatment control (cell medium) Brij58 succinate and Brij58 treated cells. Brij58 shows slight B-cell activation in elevated concentrations. Brij58 shows no B-cell activation in the concentration 0.01% (w/v) used for biologic formulations. The same applies to Brij58 succinate.

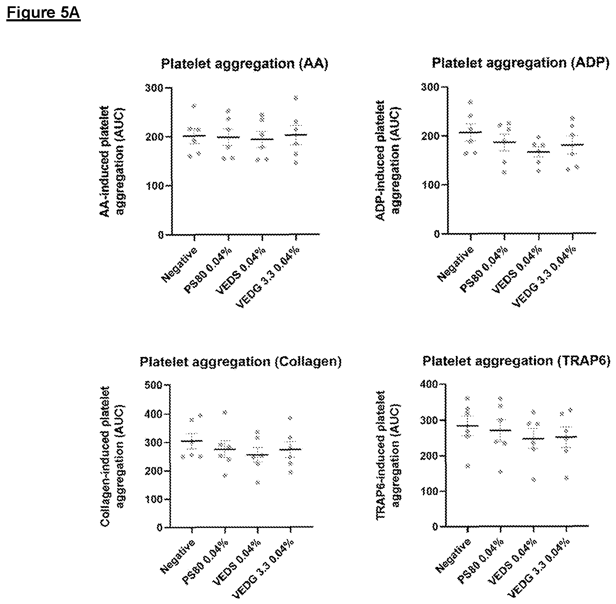

Fig. 5A shows the propensity to inhibition of platelet aggregation of PS80, VEDS and VEDG 3.3. Platelets were activated with four independent agonists. Aggregation was measured through Whole Blood Aggregometry (WBA). VEDS and VEDG 3.3 display similar platelet inhibition values as PS80.

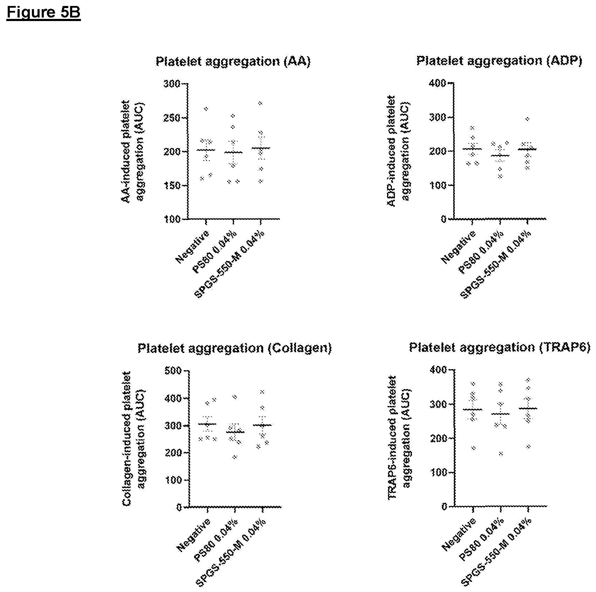

Fig. 5B shows the propensity to inhibition of platelet aggregation of PS80 and SPGS-550-M, similar to Fig. 5A. Comparison with negative control shows that SPGS-550-M has no effect on platelet aggregation.

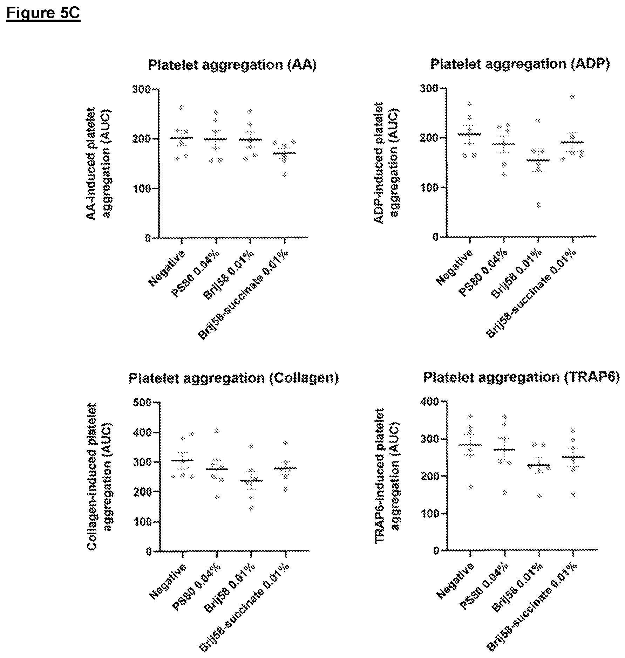

Fig. 5C shows the propensity to inhibition of platelet aggregation of PS80, Brij58 and Brij58-succinate, similar to Fig. 5A and Fig. 5B. Brij58-succinate has a lower inhibition than Brij58 on average (3/4 agonists) and closer to the levels of PS80, which is considered safe.

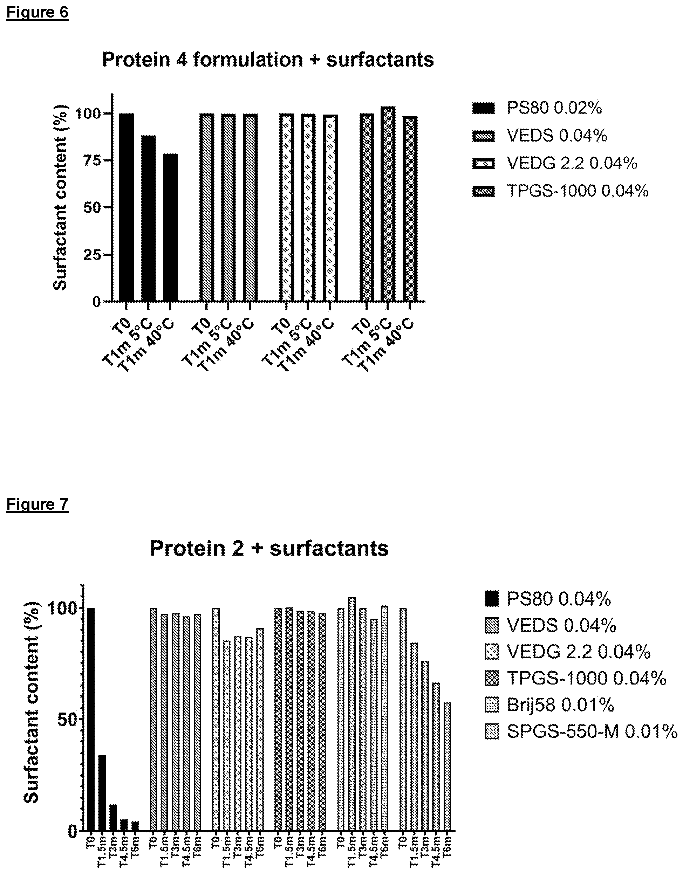

Fig. 6 shows surfactant's propensity of enzymatic degradation originating from host cell proteins (HCPs) of Protein 4. Tested surfactants: PS80, VEDS, VEDG 2.2 and TPGS-1000. PS80 is largely affected. In contrast, VEDS, VEDG 2.2 and TPGS-1000 are not affected by enzymatic degradation after one month, irrespective of the incubation temperature.
Fig. 7 shows surfactant's propensity for oxidative and hydrolytic degradation in quiescence stability studies. In said stability studies, formulations of Protein 2 comprising any of the following surfactants were used: PS80, VEDS, VEDG 2.2 and TPGS-1000, Brij58 and SPGS-550-M. Fig. 7 shows that chemical degradation is drastically affecting PS80. In contrast, VEDS, TPGS-1000 and Brij58 are not susceptible to the same degree of degradation. SPGS-550-M is susceptible to almost linear degradation.

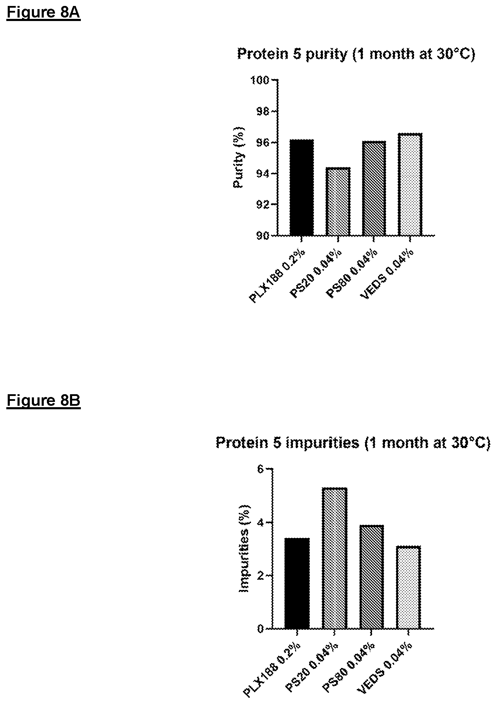

Fig. 8A shows the protein purity of formulations comprising Protein 5. The formulations contained any of the following surfactants: PLX188, PS20, PS80 and VEDS. Formulations containing PLX188, PS80 or VEDS retain a similar level of purity. The stabilization effect of PS20 is lower, i.e. lower purity is observed.
Fig. 8B shows impurities of the same formulations comprising Protein 5. Formulations containing PLX188, PS80 or VEDS retain a similar level of impurities. The impurities level of PS20 are higher, complementing purity data from Fig. 8A.

### DETAILED DESCRIPTION OF INVENTION

Surfactants are amphiphilic molecules often added to biopharmaceutical formulations in order to stabilize biologics (API) from stress encountered during manufacturing, transport, storage and administration. The exact mode of action depends on the nature of the surfactant and structural properties of the stabilized biopharmaceutical, yet, two main mechanisms have been proposed to explain their protective effect i) displacement mechanism and ii) preferential association. Without being bound by any particular theory, it is believed that when present in protein-containing formulations, amphiphilic anti-oxidant surfactants mainly exploy a displacement mechanism wherein the surfactants preferentially migrates at the interface (gas-liquid; liquid-liquid; liquid -solid) preventing protein adsorption and subsequent phenomena of protein unfolding and/or aggregation/particle formation, hence, mitigating interfacial stress that otherwise would negatively impact protein (API) quality attributes.

Surprisingly, it has been found that protein-containing formulations can be stabilized when an amphiphilic anti-oxidant surfactant is added. The present invention therefore provides protein-containing compositions that are superior in stability and a method of stabilizing a protein-containing formulation (by suppressing aggregation formation).

The present invention also provides method of stabilizing protein using an amphiphilic anti-oxidant surfactant . Furthermore, the invention also provides a surfactant of formula (I). The invention further provides the use of a surfactant of formula (I) to stabilize biological-entity containing compositions.

The invention provides stable protein-containing formulations that comprise an amphiphilic anti-oxidant surfactant.

### Definitions

The term "amphiphilic surfactant ", herein also referred to as surfactant, refers to a non-ionic surfactant like molecule having a hydrophilic head and a hydrophobic tail. Importantly, the amphiphilic surfactant in the context of the present invention is not derived from fatty acid building blocks, *e.g.,* polysorbates. In an embodiment, the amphiphilic surfactant is of a type and in an amount such that it is capable of stabilizing protein-containing compositions. The amphiphilic molecule structure can vary provided that it exhibits the desired properties of preventing protein adsorption and subsequent protein unfolding and/or aggregation/particle formation. Thus, in all aspects and embodiments of the present invention, the amphiphilic surfactant is that which is not derived from fatty acid building blocks.

The general structure of the amphiphilic surfactant is illustrated below, where R* is the hydrophilic head group and R** is the hydrophobic tail.

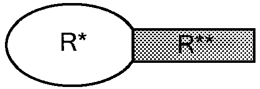

R* can be selected from any group of polar head configurations. R* can be chosen from moieties that are highly polar or hydrophilic, as well as moieties that contain alkyl chains and are less hydrophilic. The hydrophilic head of the amphiphilic anti-oxidant surfactant preferably comprises a poly(C₁-C₄alkylene) glycol group, *e.g.,* PEG group. The amphiphilic surfactant is preferably chosen from a Vitamin E analog, Sitosterol analog and polyethylene glycol alkyl ether analog, such as a poly(alkylene glycol) alkyl ether analog.

The term "amphiphilic anti-oxidant surfactant ", refers to a non-ionic surfactant like molecule having a hydrophilic head that has antioxidant or radical scavenging properties and a hydrophobic tail. In an embodiment, the amphiphilic anti-oxidant is of a type and in an amount such that it is capable of stabilizing protein-containing compositions.

The amphiphilic anti-oxidant molecule structure can vary provided that it has anti-oxidant or radical scavenging properties and exhibits the desired properties of preventing protein adsorption, subsequent protein unfolding and/or aggregation/particle formation.

The general structure of the amphiphilic anti-oxidant surfactant is illustrated below, where R* is the hydrophilic head group and R** is the hydrophobic tail.

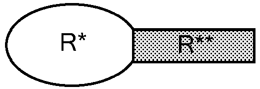

R* can be selected from any group of polar head configurations also having appropriate radical scavenging capabilities. R* can be chosen from moieties that are highly polar or hydrophilic, as well as moieties that contain alkyl chains and are less hydrophilic, provided that it provides appropriate radical scavenging functionality to protect or inhibit attack of the surfactant by an oxidant. The hydrophilic head of the amphiphilic anti-oxidant surfactant preferably comprises a poly(C₁-C₄alkylene) glycol group, *e.g.,* PEG group. The amphiphilic anti-oxidant surfactant is preferably a Vitamin E analog.

The term "not derived from fatty acid building blocks" in the context of the amphiphilic surfactants of the present disclosure means that the lipophilic core is not comprised of fatty acid moieties, *e.g.,* it is not a derivative of sorbitol esterified with fatty acids, such as polysorbates. The terms "not derived from fatty acid building blocks" and "not derived from fatty acid building block" are used synonymously. In one embodiment, the term "not derived from fatty acid building blocks" means "is not a polysorbate".

The term "Vitamin E analog" refers to compounds which have a structural motif of the vitamin E family, specifically the chroman moiety, and are amenable to various modifications in order to improve their stabilizing effects in protein-containing formulations. The phrases "Vitamin E analog" and "Vitamin E derivative" are used interchangeably.

The term "Sitosterol analog" refers to compounds which have a structural motif of the phytosterol family (*e.g.,* β-sitosterol), specifically the tetracyclic cyclopenta-α-phenanthrene ring, and are amenable to various modifications in order to improve their stabilizing effects in protein-containing formulations. The phrases "Sitosterol analog" and "Sitosterol derivative" are used interchangeably.

The term "poly(alkylene glycol) alkyl ether analog" refers to aliphatic linear alcohols with a carbon chain of 12 or more carbon atoms and terminate in a poly(alkylene glycol) group, *e.g.,* PEG group, as shown below 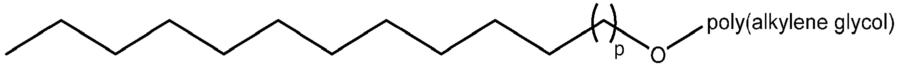 where p can be at least 1, preferably 1 to 100. Poly(alkylene glycol) alkyl ether analogs include Brij surfactants. The poly(alkylene glycol) alkyl ether analogs that may be used in the present disclosure are amenable to various modifications in order to improve their stabilizing effects in protein-containing formulations.

For example, the aliphatic linear alcohol moiety may be separated from the terminal poly(alkylene glycol) group via a linking group. The linking group (L) may form ester linkages to the aliphatic linear alcohol core structure and poly(alkylene glycol) group, for example, 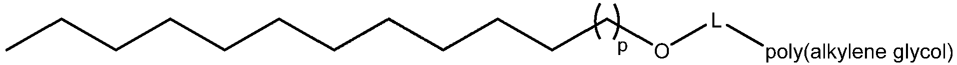 where p can be at least 1, preferably 1 to 100.

The term "dicarboxylic acid linking group" refers to a linking group X of formula -C(=O)-X-C(=O)-, wherein the linking group X is derived from a dicarboxylic acid and forms ester linkages to the core structure and poly(C₁-C₄-alkylene glycol) group of R¹. X represents a branched alkylene chain having at least 2 carbon atoms. In particular embodiments, at least one carbon atom of the branched alkylene chain of X is bonded to two C₁-C₄alkyl, *e.g.,* methyl, substituents. In particular embodiments, the branched alkylene chain of X is a C₂-C₂₀alkylene.

As used herein the term "C₁-C₁₀alkyl" refers to a straight or branched hydrocarbon chain radical consisting solely of carbon and hydrogen atoms, containing no unsaturation, having from one to ten carbon atoms, and which is attached to the rest of the molecule by a single bond. The terms "C₅-C₂₀alkyl", "C₅-C₁₅alkyl", "C₁₀-C₁₅alkyl", "C₁-C₆alkyl", and "C₁-C₄alkyl" are to be construed accordingly. Examples of C₁-C₁₀alkyl include, without limitations, methyl, ethyl, *n*-propyl, 1-methylethyl (*iso*-propyl), *n*-butyl, 1-methylpropyl (*sec*-butyl), 2-methylpropyl (*iso*-butyl), 1,1-dimethylethyl (*t*-butyl), *n*-pentyl, *n*-hexyl, *n*-heptyl, 4-heptyl, *n*-octyl, 2-*iso*propyl-3-methylbutyl, *n-*nonyl and *n*-decyl.

As used herein, the term "C₂-C₂₀alkylene" refers to a straight or branched hydrocarbon chain bivalent radical consisting solely of carbon and hydrogen atoms, containing no unsaturation, having from two to twenty carbon atoms. The terms "C₂-C₁₅alkylene", "C₂-C₁₀alkylene", "C₂-C₆alkylene", "C₃-C₁₅alkylene", "C₃-C₁₀alkylene", "C₃-C₆alkylene", "C₄-C₁₅alkylene" and "C₄-C₆alkylene" are to be construed accordingly. Examples of "C₂-C₂₀alkylene" include, without limitations, *n*-propylene, 2,2-dimethylpropylene, ethylene, 2-methylprop-2-ylene, *n*-butylene, 1-methylpropylene (*sec*-butylene), 2-methylpropylene (*iso*butylene), 1,1-dimethylethylene (*t*-butylene), *n*-pentylene, *n*-hexylene, *n*-heptylene, 4-heptylene, *n*-octylene, 2-isopropyl-3-methylbutylene, *n*-nonylene *n*-decylene and *n*-eicosylene.

The term "protein" as used herein, is used according to conventional meaning, *i.e.,* as a sequence of amino acids. Proteins are not limited to a specific length, *e.g.,* they may comprise a full length protein sequence (or peptide) or a fragment of a full length protein, and may include non-natural amino acids, *e.g.,* D-amino acids. The protein may include post-translational modifications of the protein, for example, glycosylations, acetylations, phosphorylations and the like, as well as other modifications known in the art, both naturally occurring and non-naturally occurring. Proteins useful in the formulations of the present disclosure may be prepared using any of a variety of well-known recombinant and/or synthetic techniques, illustrative examples of which are further discussed below. The protein according to the present invention is preferably a therapeutic protein and may be any protein based molecule (*e.g.,* a biologic) including, but not limited to, antibody drug conjugate, monoclonal antibody (mAb), fragment antigen binding fragment (F(ab)) or enzyme, or Fc fragment containing one or more fusion proteins.

The term "protein-containing formulation" as used herein refers to a liquid pharmaceutical composition that comprises a therapeutic protein as the active pharmaceutical ingredient (API) formulated together with one or more pharmaceutically acceptable vehicles. The protein-containing formulation is suitable for patient administration, and is used to confer a therapeutic benefit to the patient. The protein-containing formulations of the present invention are solutions (also referred to as protein-containing liquid formulations). In some embodiments, the therapeutic protein is present in a unit dose amount appropriate for administration in a therapeutic regimen. The protein-containing formulation may include, but is not limited to, a disordered protein, antibody or enzyme.

The term "stable protein-containing formulation" refers to a formulation that comprises the herein described amphiphilic surfactant. Preferably, the term "stable protein-containing formulation" refers to a formulation in which aggregation of proteins is avoided or suppressed, specifically, a formulation in which degradation such as formation of insoluble and soluble aggregates is avoided or suppressed during storage in liquid or in frozen condition.

The term "contact", "contacting", "contacted", and grammatical variations thereof, refers to placing the components of a desired reaction together under conditions suitable for carrying out the desired reaction (*e.g.,* stabilizing the protein). The term "contact" may comprise any method in which a protein is exposed to, provided with, or in which an amphiphilic surfactant is applied.

As used herein, "stabilizing" a protein-containing formulation means maintaining the structure (1°, 2°, 3° and/or 4° structure) and the function of the protein under either aqueous conditions or dried conditions, or after being frozen and/or dried and then thawed and/or rehydrated. In some embodiments, the protein may be stable at a temperature from about -80° C to about 100 °C once the protein is contacted with the amphiphilic surfactant.

The term "antibody" as used herein is used in the broadest sense and specifically covers monoclonal antibodies, polyclonal antibodies, monospecific antibodies, multispecific antibodies (*e.g.,* bispecific antibodies), modified antibodies, antibody drug conjugates and antibody fragments that exhibit the desired biological activity. The native form of an antibody is a tetramer and consists of two identical pairs of immunoglobulin chains, each pair having one light chain and one heavy chain. In each pair, the light and heavy chain variable regions (VL and VH) are together primarily responsible for binding to an antigen. The light chain and heavy chain variable domains consist of a framework region interrupted by three hypervariable regions, also called "complementarity determining regions" or "CDRs." The constant regions may be recognized by and interact with the immune system. (see, *e.g.,* Janeway et al., 2001, Immunol. Biology, 5th Ed., Garland Publishing, New York). An antibody can be of any type (*e.g.,* IgG, IgE, IgM, IgD, and IgA), class (*e.g.,* IgG1, IgG2, IgG3, IgG4, IgA1 and IgA2) or subclass. The antibody can be derived from any suitable species. In some embodiments, the antibody is of human or non-human, *e.g.,* murine origin. An antibody can be, for example, human, humanized or chimeric. The antibodies used in the present invention are not particularly limited, as long as they bind to an antigen of interest.

The term "modified antibody" as used herein refers to antibodies linked to polyethylene glycol (PEG) or various molecules such as cytotoxic agents (Farmaco. 1999 Aug 30;54(8):497-516; Cancer J. 2008 May-Jun;14(3):154-69). The "antibodies" used in the present invention also include such modified antibodies. Such modified antibodies can be prepared by chemically modifying the obtained antibodies. Such methods are already established in this field.

The term "monoclonal antibody" as used herein refers to an antibody obtained from a population of substantially homogeneous antibodies, *i.e.,* the individual antibodies comprising the population are identical except for possible naturally-occurring mutations that may be present in minor amounts. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

An "intact antibody" is one which comprises an antigen-binding variable region as well as a light chain constant domain (CL) and heavy chain constant domains, CH 1, CH 2, CH 3 and CH 4, as appropriate for the antibody class. The constant domains may be native sequence constant domains (*e.g.,* human native sequence constant domains) or amino acid sequence variant thereof.

An "antibody fragment" comprises a portion of an intact antibody, comprising the antigen binding or variable region thereof. Examples of antibody fragments include Fab, Fab', F(ab')2, and Fv fragments, diabodies, triabodies, tetrabodies, linear antibodies, single chain antibody molecules, scFv, scFv-Fc, multispecific antibody fragments formed from antibody fragment(s), a fragment(s) produced by a Fab expression library, or an epitope binding fragments of any of the above which immunospecifically bind to a target antigen (*e.g.,* a cancer cell antigen, a viral antigen or a microbial antigen).

An "antigen" is an entity to which an antibody specifically binds.

The terms "specific binding" and "specifically binds" mean that the antibody or antibody derivative will bind, in a highly selective manner, with its corresponding epitope of a target antigen and not with the multitude of other antigens. Typically, the antibody or antibody derivative binds with an affinity of at least about 1x10⁻⁷ M, and preferably 10⁻⁸ M to 10⁻⁹ M, 10⁻¹⁰ M, 10⁻¹1 M, or 10⁻¹² M and binds to the predetermined antigen with an affinity that is at least two-fold greater than its affinity for binding to a non-specific antigen (*e.g.,* BSA, casein) other than the predetermined antigen or a closely-related antigen.

The term "inhibit" or "inhibition of" means to reduce by a measurable amount, or to prevent entirely.

The term "therapeutically effective amount" refers to an amount of a protein effective to treat a disease or disorder in a mammal.

The term "antibody drug conjugate" or "ADC" as used herein refers to a compound that is linked to a cell binding agent (*i.e.,* an antibody or fragment thereof). Typically, the cell binding agent (*e.g.,* antibody) is covalently bound to the drug by a linker.

As used herein, the term "isolated" refers to a protein or peptide which is free or substantially free from other macromolecular species found in a cellular environment. A protein used in the present invention can be produced using techniques well known in the art, *e.g.,* recombinant technologies, phage display technologies, synthetic technologies or combinations of such technologies or other technologies readily known in the art.

The term "about" should be understood to permit standard variation as would be understood by those of ordinary skill in the art; and where ranges are provided, endpoints are included.

**The specification of a European patent includes the description, the claims, and, where applicable, drawings. The embodiments below form part of the description. The claims themselves appear later in the section entitled "Claims."**

### Enumerated embodiments

Embodiment 1. A compound of formula (I): 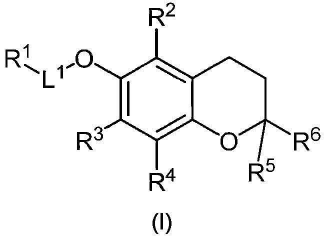 wherein for formula (I):
L¹ is -C(=O)-X-C(=O)- ;
X is a branched C₂-C₂₀alkylene R¹ is a poly(C₁-C₄-alkylene glycol) group, *e.g.,* PEG group; R², R³, R⁴ and R⁵ are each independently selected from hydrogen and C₁-C₁₀alkyl; and
R⁶ is C₅-C₂₀alkyl.

Embodiment 2. The compound according to Embodiment **1** having the formula (I), wherein R¹ is poly(C₁-C₄-alkylene glycol) group, *e.g.,* PEG group, which poly(C₁-C₄-alkylene glycol) group terminates in hydroxyl or C₁-C₆alkoxyl.

Embodiment 3. The compound according to any one of Embodiments 1 and 2 having the formula (I), wherein R¹ is -O-(CH₂-CH₂)ₘ-R^{1a}, wherein R^{1a} is selected from hydroxyl and C₁-C₆alkoxyl, and m is an integer from 1-80.

Embodiment 4. The compound according to Embodiment 3 having the formula (I), wherein m is an integer from 1-50.

Embodiment 5. The compound according to any one of Embodiments 3 and 4 having the formula (I), wherein m is an integer from 1-30.

Embodiment 6. The compound according to any one of Embodiments 1 to 5 having the formula (I), wherein R², R³, R⁴ and R⁵ are each independently C₁-C₆alkyl or formula (II), wherein R⁴ and R⁵ are each independently C₁-C₆alkyl.

Embodiment 7. The compound according to any one of Embodiments 1 to 6, having the formula (I), wherein R², R³, R⁴ and R⁵ are each independently C₁-C₆alkyl and R⁶ is C₅-C₁₅alkyl.

Embodiment 8. The compound according to any one of Embodiments 1 to 7, having the formula (I), wherein R⁶ is a branched C₁₀-C₁₅alkyl.

Embodiment 9. The compound according to any one of Embodiments 1 to 8, having the formula (I), wherein R², R³, R⁴ and R⁵ are each independently C₁-C₄alkyl.

Embodiment 10. The compound according to any one of Embodiments 1 to 9, having the formula (I), wherein R², R³, R⁴ and R⁵ are each methyl formula.

Embodiment 11. The compound according to any one of Embodiments 1 to 10, having the formula (I), wherein L¹ is a branched dicarboxylic acid linking group comprising at least one carbon atom which is bonded to two C₁-C₄alkyl substituents, *e.g.,* methyl.

Embodiment 12. The compound according to any one of Embodiments 1 to 11, having the formula (I), wherein L¹ is -C(=O)-X-C(=O)- and X is a branched C₂-C₁₅alkylene or C₂-C₁₀alkylene.

Embodiment 13. The compound according to any one of Embodiments 1 to 12, having the formula (I), wherein L¹ is-C(=O)-X-C(=O)- and X is a branched C₂-C₆Calkylene.

Embodiment 14. The compound according to any one of Embodiments 12 and 13, having the formula (I), wherein at least one carbon atom of the branched C₂-C₂₀alkylene, C₂-C₁₅alkylene, C₂-C₁₀alkylene or C₂-C₆Calkylene is bonded to two C₁-C₄alkyl groups.

Embodiment 15. The compound according to any one of Embodiments 12 to 14, having the formula (I), wherein X is a branched C₂-C₂₀alkylene, C₂-C₁₅alkylene, C₂-C₁₀alkylene or C₂-C₆Calkylene, and wherein at least one carbon atom of the branched C₂-C₂₀alkylene, C₂-C₁₅alkylene, C₂-C₁₀alkylene or C₂-C₆Calkylene is bonded to two methyl substituents.

Embodiment 16. The compound according to any one of Embodiments 12 to 15, having the formula (I), wherein X is a branched C₂-C₂₀alkylene, C₂-C₁₅alkylene, C₂-C₁₀alkylene or C₂-C₆Calkylene, and wherein one, two or three carbon atoms of the branched C₂-C₂₀alkylene, C₂-C₁₅alkylene, C₂-C₁₀alkylene or C₂-C₆Calkylene are bonded to two C₁-C₄alkyl groups, optionally to two methyl substituents.

Embodiment 17. The compound according to any one of Embodiments 12 to 16, having the formula (I), wherein X is a branched C₂-C₂₀alkylene, C₂-C₁₅alkylene, C₂-C₁₀alkylene or C₂-C₆Calkylene, and wherein one carbon atom of the branched C₂-C₂₀alkylene, C₂-C₁₅alkylene, C₂-C₁₀alkylene or C₂-C₆Calkylene is bonded to two C₁-C₄alkyl groups, optionally to two methyl substituents.

Embodiment 18. The compound according to Embodiment 1, having the formula (I), wherein:
L¹ is -C(=O)-X-C(=O)- ;
X is a branched C₂-C₂₀alkylene;
R¹ is a poly(ethylene glycol) group (PEG), which poly(ethylene glycol) group terminates in hydroxyl or C₁-C₆alkoxyl;
R², R³, R⁴ and R⁵ are each independently selected from hydrogen and C₁-C₁₀alkyl; and
R⁶ is a branched C₅-C₁₅alkyl.

Embodiment 19. The compound according to any one of Embodiments 1 and 18, having the formula (I), wherein
R¹ is -O-(CH₂-CH₂)ₘ-R^{1a}, wherein R^{1a} is selected from hydroxyl and C₁-C₆alkoxyl, and
m is an integer from 1-80, 1-50 or 1-30.

Embodiment 20. The compound according to any one of Embodiments 1, 18 and 19, having the formula (I), wherein R², R³, R⁴ and R⁵ are each independently C₁-C₆alkyl.

Embodiment 21. The compound according to any one of Embodiments 1 and 18 to 20, having the formula (I), wherein R², R³, R⁴ and R⁵ are each independently C₁-C₆alkyl, optionally methyl, and R⁶ is a branched C₅-C₁₅alkyl.

Embodiment 22. The compound according to any one of Embodiments 1 and 18 to 21, having the formula (I), wherein R⁶ is a branched C₁₀-C₁₅alkyl.

Embodiment 23. The compound according to any one of Embodiments 1 and 18 to 22, having the formula (I), wherein R², R³, R⁴ and R⁵ are each independently C₁-C₄alkyl, optionally R², R³, R⁴ and R⁵ are each methyl.

Embodiment 24. The compound according to any one of Embodiments 1 and 18 to 23, having the formula (I), wherein X is a branched C₂-C₁₅alkylene, or a branched C₃-C₁₅alkylene.

Embodiment 25. The compound according to any one of Embodiments 1 and 18 to 24, having the formula (I), wherein X is a branched C₂-C₁₀alkylene, or a branched C₃-C₁₀alkylene.

Embodiment 26. The compound according to any one of Embodiments 1 and 18 to 25, having the formula (I), wherein X is a branched C₂-C₆alkylene, or a branched C₃-C₆alkylene.

Embodiment 27. The compound according to any one of Embodiments 1 and 18 to 26, having the formula (I), wherein X is a branched C₂-C₁₅alkylene, C₂-C₁₀alkylene or C₂-C₆Calkylene, and wherein at least one carbon atom of the branched C₂-C₁₅alkylene, C₂-C₁₀alkylene or C₂-C₆Calkylene is bonded to two C₁-C₄alkyl groups.

Embodiment 28. The compound according to any one of Embodiments 1 and 18 to 27, having the formula (I), wherein X is a branched C₂-C₁₅alkylene, C₂-C₁₀alkylene or C₂-C₆Calkylene, and wherein at least one carbon atom of the branched C₂-C₁₅alkylene, C₂-C₁₀alkylene or C₂-C₆Calkylene is bonded to two methyl substituents.

Embodiment 29. The compound according to any one of Embodiments 1 and 18 to 28, having the formula (I), wherein X is a branched C₂-C₁₅alkylene, C₂-C₁₀alkylene or C₂-C₆Calkylene, and wherein one, two or three carbon atoms of the branched C₂-C₁₅alkylene, C₂-C₁₀alkylene or C₂-C₆Calkylene are bonded to C₁-C₄alkyl groups, optionally to two methyl substituents.

Embodiment 30. The compound according to any one of Embodiments 1 and 18 to 29, having the formula (I), wherein X is 2,2-dimethylpentylene or 3,3-dimethylpentylene.

Embodiment 31. The compound according to any one of Embodiments 1 to 30, wherein formula (I) is of formula (la):

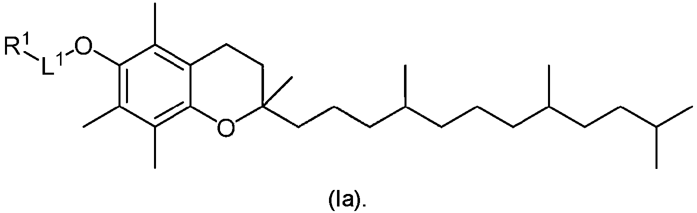

Embodiment 32. The compound according to any one of Embodiments 1 to 30, and 31, wherein formula (I) is of formula (la)-i:

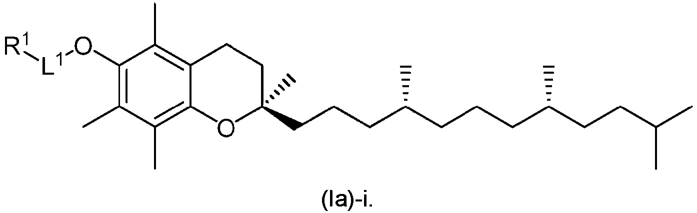

Embodiment 33. A protein-containing formulation comprising an amphiphilic surfactant, wherein the amphiphilic surfactant is present in an amount sufficient to improve the stability of the protein against aggregation.

Embodiment 34. The protein-containing formulation according to Embodiment 33, wherein the amphiphilic surfactant is an amphiphilic anti-oxidant surfactant.

Embodiment 35. The protein-containing formulation according to Embodiment 34, wherein the amphiphilic anti-oxidant surfactant is a Vitamin E analog.

Embodiment 36. The protein-containing formulation according to any one of Embodiments 33 to 35, wherein the amphiphilic surfactant comprises a poly(alkylene glycol) moiety, *e.g.,* poly(C₁-C₄alkylene glycol) moiety, *e.g.,* PEG.

Embodiment 37. The protein-containing formulation according to any one of Embodiments 33 to 36, wherein the amphiphilic surfactant is a compound having the formula (I)-i: 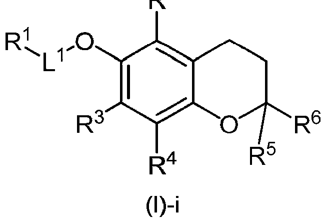 wherein:
for formula (I)-i:
L¹ is -C(=O)-X-C(=O)- ;
X is a branched C₂-C₂₀alkylene;R¹ is a poly(C₁-C₄-alkylene glycol) group, *e.g.,* PEG group, optionally wherein the poly(C₁-C₄-alkylene glycol) group terminates in hydroxyl or C₁-C₆alkoxyl; R², R³, R⁴ and R⁵ are each independently selected from hydrogen and C₁-C₁₀alkyl; and
R⁶ is C₅-C₂₀alkyl;

Embodiment 38. The protein-containing formulation according to any one of Embodiments 33 to 37, wherein the amphiphilic surfactant is a compound having the formula (I) according to any one of Embodiments 1 to 32.

Embodiment 39. The protein-containing formulation according to any one of Embodiments 33 to 38, wherein the amphiphilic surfactant is present in an amount of 4 to 20 times, optionally 4 to 10 times, optionally 4 times, 5 times, 6 times, 10 times or 20 times above the critical micelle concentration.

Embodiment 40. The protein-containing formulation according to any one of Embodiments 33 to 39, wherein the amphiphilic surfactant is present in an amount of between 0.001 and 0.5% (w/v), optionally between 0.01 and 0.1% (w/v).

Embodiment 41. The protein-containing formulation according to any one of Embodiments 33 to 40, wherein the protein is present in an amount of 1-250 mg/ml

Embodiment 42. The protein-containing formulation according to any one of Embodiments 33 to 41, wherein the protein is an antibody.

Embodiment 43. The protein-containing formulation according to Embodiment 42, wherein the antibody is an antibody-drug conjugate (ADC), monoclonal antibody (mAB) or antigen-binding fragment (Fab).

Embodiment 44. The protein-containing formulation according to Embodiment 43, wherein the monoclonal antibody is a humanized antibody or a human antibody.

Embodiment 45. The protein-containing formulation according to Embodiment 42, wherein the antibody is present in an amount of 1-250 mg/ml.

Embodiment 46. The protein-containing formulation according to any one of Embodiments 33 to 45, which is an aqueous formulation.

Embodiment 47. The protein-containing formulation according to any one of Embodiments 33 to 46, which is for subcutaneous administration.

Embodiment 48. Use of an amphiphilic surfactant as a stabilizer for a formulation comprising a protein.

Embodiment 49. The use according to Embodiment 48, wherein amphiphilic surfactant is defined according to any one of Embodiments 34 to 40.

Embodiment 50. The use according to any one of Embodiments 48 and 49, wherein the protein is defined according to any one of Embodiments 41 to 45.

Embodiment 51. A method for improving the stability of a formulation comprising a protein, the method comprising incorporating an amphiphilic surfactant in said solution, and wherein the amphiphilic surfactant component is present in an amount sufficient to improve the stability of the surfactant against aggregation.

Embodiment 52. The method of Embodiment 51, comprising the step of contacting the protein with at least one amphiphilic surfactant, to produce a liquid formulation comprising the protein and the amphiphilic surfactant, thereby stabilizing the protein.

Embodiment 53. The method of any one of Embodiments 51 and 52, wherein the amphiphilic surfactant is defined according to any one of Embodiments 34 to 40.

Embodiment 54. The method of any one of Embodiments 51 to 53, wherein the protein is defined according to any one of Embodiments 41 to 45.

Embodiment 55. A method for preventing or suppressing protein aggregation formation of a protein-containing formulation by using an amphiphilic surfactant as a stabilizer in the formulation.

Embodiment 56. The method according to Embodiment 55, wherein the method is for preventing or suppressing protein aggregation formation during storage at room temperature or during frozen storage of the protein-containing formulation.

Embodiment 57. The method according to any one of Embodiments 55 and 56, wherein the amphiphilic surfactant is defined according to any one of Embodiments 34 to 40.

Embodiment 58. The method according to any one of Embodiments 55 to 57, wherein the protein is defined according to any one of Embodiments 41 to 45.

Embodiment 59. A method of preparing a stable protein-containing formulation comprising the step of contacting the protein with at least one amphiphilic surfactant, to produce a liquid formulation comprising the protein and the amphiphilic surfactant, wherein the amphiphilic surfactant component is added in an amount sufficient to improve the stability of the surfactant against aggregation.

Embodiment 60. The method according to Embodiment 59, wherein the amphiphilic surfactant is defined according to any one of Embodiments 34 to 40.

Embodiment 61. The method according to any one of Embodiments 59 and 60, wherein the protein is defined according to any one of Embodiments 41 to 45.

Embodiment 62. A stable protein-containing formulation comprising an amphiphilic surfactant.

Embodiment 63. The protein-containing formulation according to Embodiment 62, wherein the amphiphilic surfactant is defined according to any one of Embodiments 34 to 40.

Embodiment 64. The protein-containing formulation according to any one of Embodiments 62 and 63, wherein the protein is defined according to any one of Embodiments 41 to 45.

Embodiment 65. The protein-containing formulation according to any one of Embodiments 62 to 64, wherein the formulation is defined according to any one of Embodiments 46 and 47.

Embodiment 1a. A stable protein-containing formulation comprising an amphiphilic surfactant, wherein the amphiphilic surfactant is not derived from fatty acid building blocks.

Embodiment 2a. The formulation according to embodiment 1a, wherein the amphiphilic surfactant is an amphiphilic antioxidant surfactant.

Embodiment 3a. The formulation according to embodiment 1a or 2a, wherein the amphiphilic surfactant is a Vitamin E analog.

Embodiment 4a. The formulation according to any one of the preceding embodiments 1a-3a, wherein the protein is a disordered protein or an antibody.

Embodiment 5a. The formulation according to embodiment 4a, wherein the antibody is an antibody-drug conjugate, monoclonal antibody or antigen-binding fragment.

Embodiment 6a. The formulation according to any one of embodiments 4a and 5a, wherein the antibody is present in an amount of 1-250 mg/ml.

Embodiment 7a. The formulation according to any one of embodiment 3a to 6a, wherein the Vitamin E analog, poly(alkylene glycol) alkyl ether analog.

Embodiment 8a. The formulation according to any one of the preceding embodiments 1a-7a, wherein the amphiphilic surfactant is a compound having the formula (I)-i: 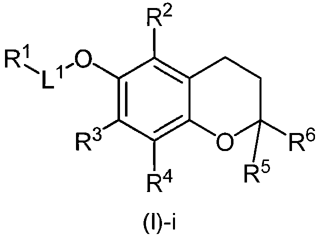 wherein:
for formula (I)-i:
L¹ is -C(=O)-X-C(=O)- ;
X is a branched C₂-C₂₀alkylene;
R¹ is a poly(C₁-C₄-alkylene glycol) group, *e.g.,* PEG group, optionally wherein the poly(C₁-C₄-alkylene glycol) group terminates in hydroxyl or C₁-C₆alkoxyl;
R², R³, R⁴ and R⁵ are each independently selected from hydrogen and C₁-C₁₀alkyl; and
R⁶ is C₅-C₂₀alkyl.

Embodiment 9a. The formulation according to embodiment 8a, wherein the amphiphilic surfactant is a compound having the formula (I), wherein
L¹ is -C(=O)-X-C(=O)- ;
X is a branched C₂-C₂₀alkylene;
R¹ is a PEG group, which PEG group terminates in hydroxyl or C₁-C₆alkoxyl;
R², R³, R⁴ and R⁵ are each independently selected from hydrogen and C₁-C₁₀alkyl; and
R⁶ is C₅-C₂₀alkyl.

Embodiment 10a. The formulation according to any one of embodiments 1a to 9a, which is for subcutaneous administration.

Embodiment 11a. Use of an amphiphilic surfactant as a stabilizer for a formulation comprising a protein, wherein the amphiphilic surfactant is not derived from fatty acid building blocks.

Embodiment 12a. The use according to embodiment 11a, wherein amphiphilic surfactant is defined according to any one embodiments 1a to 3a, and 7a to 9a.

Embodiment 13a. The use according to any one of embodiments 11a and 12a, wherein the protein is defined according to any one of embodiments 4a to 6a.

Embodiment 14a. A method for preventing or suppressing protein aggregation formation of a protein-containing formulation by using an amphiphilic surfactant as a stabilizer in the formulation, wherein the amphiphilic surfactant is not derived from fatty acid building blocks.

Embodiment 15a. The method according to embodiment 14a, wherein the amphiphilic surfactant is defined according to any one of embodiments 1a to 3a, and 7a to 9a.

Embodiment 16a. The method of any one of embodiments 14a and 15a, wherein the protein is defined according to any one of embodiment 4a to 6a.

Embodiment 17a. A surfactant of formula (I): 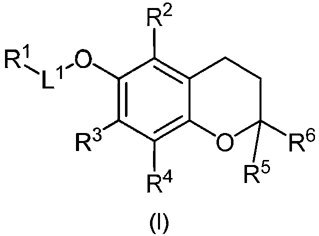 wherein for formula (I):
L¹ is -C(=O)-X-C(=O)- ;
X is a branched C₂-C₂₀alkylene R¹ is a poly(C₁-C₄-alkylene glycol) group, *e.g.,* PEG group;
R², R³, R⁴ and R⁵ are each independently selected from hydrogen and C₁-C₁₀alkyl; and
R⁶ is C₅-C₂₀alkyl;
wherein for formula (II):
   L¹ is a branched dicarboxylic acid linking group;
   R¹ is a poly(C₁-C₄-alkylene glycol) group, *e.g.,* PEG group;
   R⁴ and R⁵ are each independently selected from hydrogen and C₁-C₁₀alkyl; and
   R⁶ is C₅-C₂₀alkyl; and
   wherein for formula (III):
      L¹ is a straight chain or branched dicarboxylic acid linking group;
      R¹ is a poly(C₁-C₄-alkylene glycol) group, *e.g.,* PEG group; and
      n is 12 to 100, *e.g.,* 12 to 80.

### Surfactant of Formula (I)

The present invention provides a surfactant of formula (I): 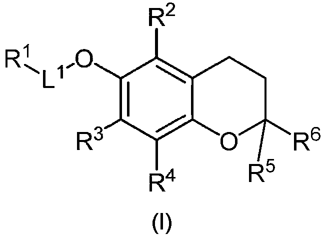 wherein:
L¹ is branched dicarboxylic acid linking group;
R¹ is a poly(C₁-C₄-alkylene glycol) group, *e.g.,* PEG group;
R², R³, R⁴ and R⁵ are each independently selected from hydrogen and C₁-C₁₀alkyl; and
R⁶ is C₅-C₂₀alkyl;
wherein for formula (II):
   L¹ is -C(=O)-X-C(=O)- ;
   R¹ is a poly(C₁-C₄-alkylene glycol) group, *e.g.,* PEG group, optionally wherein the poly(C₁-C₄-alkylene glycol) group terminates in hydroxyl or C₁-C₆alkoxyl;
   R², R³, R⁴ and R⁵ are each independently selected from hydrogen and C₁-C₁₀alkyl; and
   R⁶ is C₅-₂₀alkyl.

The poly(C₁-C₄-alkylene glycol) group may have an average molecular weight in the range of about 100 to about 10,000 g/mol, especially in the range of about 200 to about 5,000 g/mol, in particular in the range of about 250 to 2500 g/mol, about 400 to about 2,000 g/mol or about 500 to 1500 g/mol, such as about 550, about 750 or about 1000 g/mol.

The poly(C₁-C₄-alkylene glycol) group, *e.g.,* poly(ethylene) glycol, poly(propylene) glycol, of residue R¹ is attached to the oxygen atom of the core structure via a linking group L¹. The linking group is a branched dicarboxylic acid linking group. Such linking groups form ester linkages to the core structure and poly(C₁-C₄-alkylene glycol) In particular, the branched dicarboxylic acid linking group of L¹ comprises at least one carbon atom bonded to two C₁-C₄alkyl groups, preferably to two methyl substituents. Exemplary linking groups including branched dicarboxylic acid derivatives, *e.g.,* having 2 to 20 carbon atoms, are 2,2-dimethylsuccinic acid, 2,2-dimethylglutaric acid and 3,3-dimethylglutaric acid.

It has been found that when at least one carbon atom of the branched dicarboxylic acid linking group is bonded to two C₁-C₄alkyl groups, *e.g.,* methyl, the branched dicarboxylic acid linking group affords even better protection of the surfactants against chemical and enzymatic degradation of the neighboring ester groups.

In certain embodiments, the poly(C₁-C₄-alkylene glycol) group of R¹ further comprises a terminal group attached to the end of the polyethylene glycol group. This terminal group in particular is attached to the polyethylene glycol group via an ether, ester or amide linkage, preferably the terminal group in particular is attached to the polyethylene glycol group via an ether linkage. The terminal group especially is a hydroxyl or C₁-C₆alkoxyl group, in particular a methoxyl (-OCH₃) group.

R², R³, R⁴ and R⁵ are independently hydrogen or C₁-C₁₀alkyl, in particular hydrogen, methyl or ethyl, especially hydrogen or methyl. In certain embodiments, R⁵ is methyl. In certain embodiments, R⁴ is methyl. In certain embodiments, R⁴ and R⁵ are methyl. In further embodiments, R² and R³ are independently hydrogen or methyl. In specific embodiments, R⁴ and R⁵ are methyl and R² and R³ are independently hydrogen or methyl. In certain embodiments where the surfactant has formula (I), R², R³, R⁴ and R⁵ are all methyl. In certain embodiments where the surfactant has formula (II), R⁴ and R⁵ are both methyl.

R⁶ is C₅-C₂₀alkyl. R⁶ may be linear or branched and is unsubstituted. In particular, R⁶ is branched.

In embodiments where the surfactant has formula (I), R⁶ may be C₅-C₂₀alkyl, especially C₁₀-C₁₅alkyl, in particular branched C₁₀-C₁₅alkyl. In specific embodiments, R⁶ is branched C₁₅alkyl, in particular 4,8,12-trimethyltridecyl. In certain embodiments, the carbon atom attached to R⁵ and R⁶ has the *R* configuration.

### Methods of use

Any references to methods of treatment by therapy or surgery or *in vivo* diagnosis methods of this description is to be interpreted as a reference to compounds, pharmaceutical compositions and medicaments of the present invention for use in those methods.

The present invention suppresses, inhibits or prevents aggregation of proteins in liquid formulations. Thereby, the stability of the protein-containing formulation is increased and the amount of unwanted aggregates and/or degradation products is reduced or prevented. In view of this, the present invention in a further aspect provides the use of an amphiphilic surfactant as a stabilizer for protein-containing formulations. Preferably, the amphiphilic surfactant is a Vitamin E analog, Sitosterol analog or poly(alkylene glycol) alkyl ether analog.

In certain embodiments, the amphiphilic surfactant is an amphiphilic anti-oxidant surfactant. In particular, the amphiphilic anti-oxidant surfactant is a Vitamin E analog.

According to the invention, the amphiphilic surfactant is a compound having the formula (I)-i: 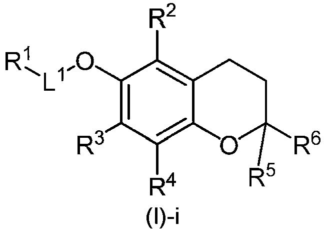 wherein the groups L¹, X, R² to R⁶ have the afore meaning.

In embodiments where the surfactant is a Vitamin E analog, *e.g.,* has formula (I)-i or (I), the surfactant especially is derived from vitamin E, in particular from tocopherol, especially from α-tocopherol. The surfactant can be any amphiphilic surfactant which is present in an amount sufficient to improve the stability of the protein against aggregation. In certain embodiments, the amphiphilic surfactant has a molecular weight of 10,000 g/mol or less, in particular 7,500 g/mol or less, 5,000 g/mol or less, 3,000 g/mol or less, 2,500 g/mol or less or even 2,000 g/mol or less.

Certain examples of the surfactant include tocopherol polyethylene glycol succinates (TPGS), in particular DL-α-tocopherol polyethylene glycol succinates such as TPGS-750-M, TPGS-1000, TPGS-1500, TPGS-400, TPGS-1100-M, TPGS-2000, TPGS-860-oleate, TPGS-PEG-PPG-PEG-1100 and TPGS-PPG-PEG-70-butyl, and DL-α-tocopherol polypropylene glycol succinates such as TPPG-1000 and TPPG-1000-butyl; and polyethylene glycol α-tocopherol diester of sebacic acid (PTS) such as PTS-600. In certain embodiments, the Vitamin E analog is of formula (I) according to any one of embodiments 1 to 30, 52, and 53.

The surfactant compounds having a formula (I)-i comprise a poly(C₁-C₄-alkylene glycol) group in residue R¹. The poly(C₁-C₄-alkylene glycol) group, *e.g.,* PEG, may have an average molecular weight in the range of about 100 to about 10,000 g/mol, especially in the range of about 200 to about 5,000 g/mol, in particular in the range of about 250 to 2500 g/mol, about 400 to about 2,000 g/mol or about 500 to 1500 g/mol, such as about 550, about 750 or about 1000 g/mol.

The poly(C₁-C₄-alkylene glycol) group of residue R¹ is attached to the oxygen atom of the core structure via a linking group L¹. The linking group is a branched dicarboxylic acid which forms ester linkages to the core structure and poly(C₁-C₄-alkylene glycol) group. In particular, the branched dicarboxylic acid linking group of L¹ comprises at least one carbon atom bonded to two C₁-C₄alkyl groups, preferably to two methyl substituents. Exemplary linking groups of branched dicarboxylic acid groups have 2 to 20 carbon atoms, such as 2,2-dimethylsuccinic acid, 2,2-dimethylglutaric acid and 3,3-dimethylglutaric acid.

In certain embodiments, R¹ further comprises a terminal group attached to the end of the poly(C₁-C₄-alkylene glycol) group. This terminal group in particular is attached to the poly(C₁-C₄-alkylene glycol) group via an ether, ester or amide linkage, preferably the terminal group in particular is attached to the poly(C₁-C₄-alkylene glycol) group via an ether linkage. The terminal group especially is a hydroxyl or C₁-C₆alkoxyl group, in particular a methoxyl (-OCH₃) group.

R², R³, R⁴ and R⁵ are independently hydrogen or C₁-C₁₀alkyl, in particular hydrogen, methyl or ethyl, especially hydrogen or methyl. In certain embodiments, R⁵ is methyl. In certain embodiments, R⁴ is methyl. In certain embodiments, R⁴ and R⁵ are methyl. In further embodiments, R² and R³ are independently hydrogen or methyl. In specific embodiments, R⁴ and R⁵ are methyl and R² and R³ are independently hydrogen or methyl. In certain embodiments where the surfactant has formula (I)-i, R², R³, R⁴ and R⁵ are all methyl.

R⁶ is C₅-C₂₀alkyl. R⁶ may be linear or branched and is unsubstituted. In particular, R⁶ is branched.

In embodiments where the surfactant has formula (I)-i, R⁶ may be C₅-C₂₀alkyl, especially C₁₀-C₁₅alkyl, in particular branched C₁₀-C₁₅alkyl. In specific embodiments, R⁶ is branched C₁₅alkyl, in particular 4,8,12-trimethyltridecyl. In certain embodiments, the carbon atom attached to R⁵ and R⁶ has the *R* configuration.

In a further aspect, the present invention provides a method for improving the stability of a formulation comprising a protein, the method comprising incorporating an amphiphilic surfactant in said solution, and wherein the amphiphilic surfactant component is present in an amount sufficient to improve the stability of the surfactant against aggregation. In certain embodiments, the method comprises the step of contacting the protein with at least one amphiphilic surfactant, to produce a liquid formulation comprising the protein and the amphiphilic surfactant, thereby stabilizing the protein. All of the aforementioned embodiments in this section relating to the use of an amphiphilic surfactant as a stabilizer for protein-containing formulations, are equally applicable to the aforementioned method.

In a further aspect, the present invention provides a method for preventing or suppressing protein aggregation formation of a protein-containing formulation by using an amphiphilic anti-oxidant surfactant as a stabilizer in the formulation, in particular, for preventing or suppressing protein aggregation formation, *e.g.,* during frozen storage or liquid storage of the formulation. All the aforementioned embodiments in this section relating to the use of an amphiphilic surfactant as a stabilizer for protein-containing formulations, are equally applicable to the aforementioned method.

Furthermore, the present invention provides the use of an amphiphilic surfactant in manufacturing a stable protein-containing formulation. All the aforementioned embodiments in this section relating to the use of an amphiphilic surfactant as a stabilizer for protein-containing formulations, are equally applicable to the aforementioned use.

### Protein-containing formulations comprising an amphiphilic surfactant

The present invention provides a protein-containing formulation comprising an amphiphilic surfactant, wherein the amphiphilic surfactant is present in an amount sufficient to improve the stability of the protein against aggregation. The amphiphilic surfactant used in the protein-containing formulations of the present invention is not derived from fatty acid building blocks, e.g., polysorbates.

The present invention therefore provides a stable protein-containing formulation comprising an amphiphilic surfactant.

The protein may be an antibody (*e.g.,* ADC, mAb, Fab), intrinsically disordered protein or an enzyme. The protein is preferably an antibody.

The antibodies used in the present invention are not particularly limited, as long as they bind to an antigen of interest.

The immunoglobulin class of antibodies that may be used in the formulations of the present invention is not particularly limited; and the class may be any class, including IgG such as IgG1, IgG2, IgG3, and IgG4, IgA, IgD, IgE, and IgM.

The antibodies that may be used in the formulations of the present invention may also include not only whole antibodies but also antibody fragments such as Fv, Fab, and F(ab)2, and minibodies (low molecular weight antibodies) such as monovalent or bivalent single-chain Fv that result from linking antibody variable regions via a linker such as peptide linker (scFv, sc(Fv)2 , diabodies such as scFv dimer, etc).

The above-described antibodies which may be used in the present invention can be prepared by methods known to those skilled in the art.

In some embodiments, the antibody is a monoclonal antibody. The monoclonal antibodies which may be used in the present invention include not only those derived from animals such as humans, mice, rats, hamsters, rabbits, sheep, camels, and monkeys, but also artificially modified gene recombinant antibodies such as chimeric antibodies, humanized antibodies, and bispecific antibodies. The antibodies may also include gene recombinant antibodies that result from artificially modifying the antibody constant regions to alter the physical properties of the antibody molecule (specifically, alteration of the isoelectric point (pl), improvement of the affinity for Fc receptor, etc) for the purpose of improving the blood persistence and in vivo pharmacokinetics.

In some embodiments, the antibody is an antigen binding fragment. For example, the antigen binding fragment may comprise one or more of a single chain variable fragment (scFv), a single domain antibody, a single chain antibody, and the heavy and/or light chain of a Fab. The antigen binding fragment may also be a Fab, such as a Fab comprising a modified light chain constant region or an unmodified Fab. In some embodiments, the protein is an antigen binding fragment. Fab or other antigen binding compound that may be used in the present invention may be derived from a single copy or clone (*e.g.,* a monoclonal antibody (mAb)), including any eukaryotic, prokaryotic, or phage clone. The antibody, Fab or other antigen binding compound, or a nucleic acid encoding the same, may be provided in isolated form.

Monoclonal antibody-producing hybridomas can be prepared by the conventional methods described below. Specifically, immunization is carried out by a conventional immunization method using a desired antigen or cells expressing the desired antigen as a sensitizing antigen. The prepared immunocytes are fused with known parental cells by a conventional cell fusion method. The fused cells are screened for monoclonal antibody-producing cells (hybridomas) by conventional screening methods. Hybridomas can be generated, for example, according to the method of Milsteinet al. ( Kohler, G. and Milstein, C., Methods Enzymol. (1981) 73:3-46). When an antigen has low immunogenicity, immunization can be performed using the antigen linked to immunogenic macromolecules such as albumin.

Alternatively, it is possible to use gene recombinant antibodies produced using gene recombination techniques in which antibody genes are cloned from hybridomas and inserted into appropriate vectors, and the resulting vectors are introduced into hosts (see, for example, Carl, A. K. Borrebaeck, James, W. Larrick, Therapeutic Monoclonal Antibodies, Published in the United Kingdom by Macmillan Publishers, 1990 ). Specifically, cDNAs for antibody variable regions (V regions) are synthesized from the mRNAs of hybridomas using reverse transcriptase. When a DNA encoding an antibody V region of interest is obtained, the DNA is linked to a DNA encoding a desired antibody constant region (C regions). The resulting construct is inserted into an expression vector. Alternatively, the antibody V region-encoding DNA may be inserted into an expression vector carrying the DNA of the antibody C region. The resulting construct is inserted into an expression vector so that it is expressed under the control of an expression regulatory region, for example, enhancer and promoter. Then, host cells are transformed with the expression vector to express the antibody.

In the present invention, artificially modified gene recombinant antibodies such as chimeric and humanized antibodies can be used to reduce heterologous antigenicity against human. Such modified antibodies can be produced using known methods. A chimeric antibody is an antibody having the heavy-chain and light-chain variable regions of an antibody from a nonhuman mammal such as mouse, and the heavy-chain and light-chain constant regions of a human antibody. The chimeric antibody can be produced by linking a DNA encoding the variable regions of a mouse antibody to a DNA encoding the constant regions of a human antibody, inserting the ligate into an expression vector, and then introducing the vector into a host for expression.

A humanized antibody is also referred to as reshaped human antibody, and is obtained by substituting the complementarity determining region (CDR) of a human antibody for the complementarity determining region of an antibody derived from a nonhuman mammal, for example, mouse. Conventional gene recombination techniques are known. Specifically, a DNA sequence is designed to have a mouse antibody CDR linked to a human antibody framework (FR) region, and is synthesized by PCR using several oligonucleotides prepared to have overlapping regions at their ends. The obtained DNA is ligated to a DNA encoding a human antibody constant region and then inserted into an expression vector. The expression vector is introduced into a host to produce the humanized antibody (see, European Patent Application Publication No. EP 239400 and WO 96/02576 ). The CDR-linked human antibody FR is selected so that the complementarity determining region forms a preferable antigen-binding domain. Amino acids in the framework region of the antibody variable region can be substituted as required so that the complementarity determining region of the reshaped human antibody forms a suitable antigen-binding domain ( Sato, K. et al., Cancer Res. (1993) 53, 851-856 ).

Methods for obtaining human antibodies are also known. For example, desired human antibodies with antigen-binding activity can be obtained by sensitizing human lymphocytes with an antigen of interest or cells expressing an antigen of interest in vitro; and fusing the sensitized lymphocytes with human myeloma cells such as U266 (see Japanese Patent Application Kokoku Publication No. (JP-B) H01-59878 (examined, approved Japanese patent application published for opposition)). Alternatively, desired human antibodies can also be obtained by immunizing transgenic animals having the entire repertoire of human antibody genes with an antigen (see WO 93/12227 , WO 92/03918 , WO 94/02602 , WO 94/25585 , WO 96/34096 , and WO 96/33735 ). Furthermore, techniques for obtaining human antibodies by panning with a human antibody library are known. For example, the variable regions of human antibodies can be expressed as single-chain antibodies (scFvs) on the surface of phages using a phage display method, and then phages that bind to the antigen can be selected. The genes of selected phages can be analyzed to determine the DNA sequences that encode the variable regions of human antibodies that bind to the antigen. When the DNA sequences of scFvs that bind to the antigen are identified, appropriate expression vectors carrying these sequences can be constructed to obtain human antibodies. Such methods are already well known. See WO 92/01047 , WO 92/20791 , WO 93/06213 , WO 93/11236 , WO 93/19172 , WO 95/01438 , and WO 95/15388 . The antibodies used in the present invention also include such human antibodies.

When the antibody genes are isolated and introduced into appropriate hosts to produce antibodies, hosts and expression vectors can be used in appropriate combinations. When eukaryotic cells are used as a host, animal cells, plant cells, and fungal cells can be used. The animal cells include: (1) mammalian cells such as CHO, COS, myeloma, baby hamster kidney (BHK), HeLa, and Vero cells; (2) amphibian cells such as Xenopus oocytes; and (3) insect cells such as sf9, sf21, and Tn5. Known plant cells include cells derived from genus Nicotiana such as Nicotiana tabacum, which can be cultured as a callus. Known fungal cells include yeasts such as genus Saccharomyces, for example Saccharomyces cerevisiae, and filamentous fungi such as genus Aspergillus, for example Aspergillus niger. When using prokaryotic cells, production systems using bacterial cells can be used. Known bacterial cells include Escherichia coli (E. coli) and Bacillus subtilis. The antibodies can be obtained by introducing the antibody genes of interest into these cells by transformation and then culturing the transformed cellsin vitro.

There are known techniques for substituting amino acids in antibodies to improve antibody activities, physical properties, pharmacokinetics, safety, and such. Examples of such techniques are described below. The antibodies of the present invention also include those having such amino acid substitutions.

Techniques are reported for substituting amino acids in the IgG antibody variable regions, and include humanization ( Tsurushita N, Hinton PR, Kumar S., Design of humanized antibodies: from anti-Tac to Zenapax., Methods. 2005 May;36(1):69-83 ); affinity maturation to enhance the binding activity via amino acid substitution in the complementarity determining region (CDR) ( Rajpal A, Beyaz N, Haber L, Cappuccilli G, Yee H, Bhatt RR, Takeuchi T, Lerner RA, Crea R., A general method for greatly improving the affinity of antibodies by using combinatorial libraries., Proc Natl Acad Sci USA. 2005 Jun 14;102(24):8466-71 ); and improvement of physicochemical stability via amino acid substitution in the framework (FR) (Ewert S, Honegger A, Pluckthun A., Stability improvement of antibodies for extracellular and intracellular applications: CDR grafting to stable frameworks and structure-based framework engineering., Methods. 2004 Oct;34(2):184-99 . Review). There are also known techniques for enhancing antibody-dependent cell cytotoxicity (ADCC) and complement-dependent cytotoxicity (CDC) by substituting amino acids in IgG antibody Fc domain ( Kim SJ, Park Y, Hong HJ., Antibody engineering for the development of therapeutic antibodies., Mol Cells. 2005 Aug 31;20(1):17-29 . Review). Furthermore, in addition to techniques for enhancing the effector functions, there are reports published on techniques for improving the antibody half-life in blood by substituting amino acids in Fc ( Hinton PR, Xiong JM, Johlfs MG, Tang MT, Keller S, Tsurushita N., An engineered human IgG1 antibody with longer serum half-life., J Immunol. 2006 Jan 1;176(1):346-56 ; Ghetie V, Popov S, Borvak J, Radu C, Matesoi D, Medesan C, Ober RJ, Ward ES., Increasing the serum persistence of an IgG fragment by random mutagenesis., Nat Biotechnol. 1997 Jul;15(7):637-40 ). Another known technique includes amino acid substitution technique to control the isoelectric point (pl) of an antibody for the purpose of improving the blood persistence or in vivo pharmacokinetics, specifically, a technique for modifying amino acid residues exposed on the surface of an antibody to control the pl of the antibody ( WO 07/114319). Various techniques to substitute amino acids in the constant regions for the purpose of improving the physical properties of an antibody are also known ( WO 09/41613 ).

In some embodiments, the protein is a disordered protein. When disordered protein genes are isolated and introduced into appropriate hosts to produce disordered proteins, hosts and expression vectors can be used in appropriate combinations. When eukaryotic cells are used as a host, animal cells, plant cells, and fungal cells can be used. The animal cells include: (1) mammalian cells such as CHO, COS, myeloma, baby hamster kidney (BHK), HeLa, and Vero cells; (2) amphibian cells such as Xenopus oocytes; and (3) insect cells such as sf9, sf21, and Tn5. Known plant cells include cells derived from genus Nicotiana such as Nicotiana tabacum, which can be cultured as a callus. Known fungal cells include yeasts such as genus Saccharomyces, for example Saccharomyces cerevisiae, and filamentous fungi such as genus Aspergillus, for example Aspergillus niger.

The concentration of the protein, *e.g.,* antibody (*e.g.,* ADC, mAb, Fab), intrinsically disordered protein or enzyme in the protein-containing formulation of the present invention is not particularly limited. The concentration of the protein is preferably 5 mg/ml or more, 10 mg/ml or more, 25 mg/ml or more, 50 mg/ml or more, more preferably 100 mg/ml or more, even more preferable 120 mg/ml or more, still more preferably 150 mg/ml or more, and yet more preferably 180 mg/ml or more. The upper limited the antibody concentration in a formulation of the present invention is not particularly limited; however, the limit is generally 300 mg/ml. The concentration of the protein may be from 1-300 mg/ml, *e.g.,* from 1-250 mg/ml.

The stability of the protein formulations of the present invention can comprise evaluating the chemical stability, physical stability or functional stability of the protein and/or surfactant. This can be measured, for example, by using forced degradation assays. The formulations of the present invention typically exhibit high levels of protein stability. Thus, the proteins within the formulations can retain an acceptable degree of chemical structure or biological function after storage under exemplary conditions as defined herein. A formulation may be stable even though the protein contained therein does not maintain 100% of its chemical structure or biological function after storage for a defined amount of time. Under certain circumstances, maintenance of about 90%, about 95%, about 96%, about 97%, about 98%, or about 99% of a protein's structure or function after storage for a defined amount of time may be regarded as "stable".

Stability can be measured, inter *alia,* by determining the percentage of native protein that remains in the formulation after storage for a defined amount of time at a defined temperature. The percentage of native protein can be determined by, inter alia, size exclusion chromatography, (*e.g.,* size exclusion high performance liquid chromatography (SE-HPLC)), such that native means non-aggregated and non-degraded. An "acceptable degree of stability", as used herein, means that at least 90% of the native form of the protein can be detected in the formulation after storage for a defined amount of time at a given temperature. In certain embodiments, at least about 90%, 91%, 92%, 93%, 94%, 95%, 96%, 97%, 98%, 99% or 100% of the native form of the protein can be detected in the formulation after storage for a defined amount of time at a defined temperature. The defined amount of time after which stability is measured can be at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or more.

The temperature at which the pharmaceutical formulation may be stored when assessing stability can be any temperature from about -80 °C to about 45 °C, e.g., storage at about -80 °C, about -30 °C, about -20 °C, about 0 °C, about 5 °C, about 25 °C, about 35 °C, about 37 °C, or about 45 °C. For example, the pharmaceutical formulations may be deemed stable if after 6 months or storage at 5°C, greater than about 95%, 96%, 97%, or 98% of native protein is detected by SE-HPLC. A pharmaceutical formulation may also be deemed stable if after 6 months of storage at room temperature, greater than about 94%, 95%, 96%, 97%, or 98% of native protein is detected by SE-HPLC. The pharmaceutical formulation may also be deemed stable if after 28 days of storage at 45 °C, greater than about 91%, 92%, 93%, 94%, 95%, 96%, 97%, or 98% of native protein is detected by SE-HPLC. The pharmaceutical formulation may also be deemed stable if after three months of storage at -20 °C, greater than about 96%, 97% or 98% of native protein is detected by SE-HPLC. The pharmaceutical formulation may also be deemed stable if after three months of storage at -30 °C, greater than about 96%, 97% or 98% of native protein is detected by SE-HPLC. The pharmaceutical formulation may also be deemed stable if after three months of storage at -80 °C, greater than about 96%, 97% or 98% of native protein is detected by SE-HPLC.

Stability can be also be measured, inter *alia,* by determining the concentration of particles present in the protein-containing formulation. The concentration of particles can be determined by, inter *alia,* micro-flow-imaging (MFI) or light obscuration (LO). There are many proteinaceous and non-proteinaceous particle classifications depending on criteria such as size, source, or composition. While from an analytical point of view, classification by size is the major attribute to characterize particles. Particles are defined as material with a size above 0.1 µm and are further classified into sub visible (0.1-100 µm) and visible particles (above 100 µm). Submicron particles (0.1-1 µm) are a subgroup of sub visible. Protein particles are a subset of protein aggregates. In certain embodiments, less than about 6000 particles ≥10µm and/or less than 600 particles ≥25µm per total volume of 100mL or less (small volume parenteral (SVP)) as measured by light obscuration can be detected in the formulation after storage for a defined amount of time at a defined temperature as defined in USP <787> and USP <788>.

In certain embodiments, less than about 25 particles per mL ≥10µm and/or less than 3 particles per mL ≥25µm for total volume of 100mL or more (large volume parenteral (LVP)) as measured by light obscuration can be detected in the formulation after storage for a defined amount of time at a defined temperature as defined in USP <787> and USP <788>.

The defined amount of time after which stability is measured can be at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or more.

The temperature at which the pharmaceutical formulation may be stored when assessing stability can be any temperature from about -80 °C to about 45 °C, *e.g.,* storage at about -80 °C, about -30 °C, about -20 °C, about 0 °C, about 5 °C, about 25 °C, about 35 °C, about 37 °C, or about 45 °C. For example, the pharmaceutical formulations may be deemed stable if after 6 months of storage at 5°C, less than about 6000, 5000, 4000, 3000, 2000, 1000 particles ≥10µm and/or less than about 600, 500, 400, 300, 200, 100 particles ≥25µm at a volume of 100mL are detected by light obscuration. A pharmaceutical formulation may also be deemed stable if after 6 months of storage at room temperature, less than about 6000, 5000, 4000, 3000 particles ≥10µm and/or less than about 600, 500, 400, 300, particles ≥25µm at a volume of 100mL are detected by light obscuration. The pharmaceutical formulation may also be deemed stable if after 28 days of storage at 45 °C, less than about 6000, 5000, 4000, particles ≥10µm and/or less than about 600, 500, 400 particles ≥25µm at a volume of 100mL are detected by light obscuration. The pharmaceutical formulation may also be deemed stable if after three months of storage at -20 °C, less than about 6000, 5000, 4000, 3000, 2000, 1000 particles ≥10µm and/or less than about 600, 500, 400, 300, 200, 100 particles ≥25µm at a volume of 100mL are detected by light obscuration. The pharmaceutical formulation may also be deemed stable if after three months of storage at -30 °C, less than about 6000, 5000, 4000, 3000 particles ≥10µm and/or less than about 600, 500, 400, 300, particles ≥25µm at a volume of 100mL are detected by light obscuration. The pharmaceutical formulation may also be deemed stable if after three months of storage at -80 °C, less than about 6000, 5000, 4000, 3000, 2000, 1000 particles ≥10µm and/or less than about 600, 500, 400, 300, 200, 100 particles ≥25µm at a volume of 100mL volume are detected by light obscuration.

Other methods may be used to assess the stability of the formulations of the present invention such as, e.g., differential scanning calorimetry (DSC) to determine thermal stability, controlled agitation to determine mechanical stability, and absorbance at about 350 nm or about 405 nm to determine solution turbidity. For example, a formulation of the present invention may be considered stable if, after 6 or more months of storage at about 5°C to about 25°C, the change in optical density at 405 nm (OD405) of the formulation is less than about 0.05 (*e.g.,* 0.04, 0.03, 0.02, 0.01, or less) from the OD405 of the formulation at time zero. Measuring the biological activity or binding affinity of the protein to its target may also be used to assess stability. For example, a formulation of the present invention may be regarded as stable if, after storage at *e.g.,* 5°C, 25°C, 45°C, etc. for a defined amount of time (*e.g.,* from 1 to 12 months), the protein contained within the formulation binds to its target with an affinity that is at least 90%, 95%, or more of the binding affinity of the protein prior to said storage. Binding affinity may be determined by *e.g.,* ELISA or Surface Plasmon Resonance. Biological activity may be determined by a protein activity assay, such as *e.g.,* contacting a cell that expresses the protein with the formulation comprising the a protein. The binding of the protein to such a cell may be measured directly, such as *e.g.,* via FACS analysis. Alternatively, the downstream activity of the protein system may be measured in the presence of the protein, and compared to the activity of the protein system in the absence of protein. Additional methods for assessing the stability of a protein in formulation are demonstrated in the Examples presented herein.

The surfactant in the formulation of the present invention can be any surfactant which is present in an amount sufficient to improve the stability of the protein against aggregation. In certain embodiments, the amphiphilic surfactant has a molecular weight of 10,000 g/mol or less, in particular 7,500 g/mol or less, 5,000 g/mol or less, 3,000 g/mol or less, 2,500 g/mol or less or even 2,000 g/mol or less.

In certain embodiments, the hydrophilic part of the amphiphilic surfactant present in the formulation comprises a polyalkylene glycol moiety, especially a polyethylene glycol moiety or a polypropylene glycol moiety. The polyalkylene moiety, especially the polyethylene glycol moiety, may have an average molecular weight in the range of about 100 to about 10,000 g/mol, especially in the range of about 300 to about 3,000 g/mol, in particular in the range of about 400 to about 2,000 g/mol. Certain examples of the surfactant comprising a polyalkylene glycol moiety include tocopherol polyethylene glycol succinates (TPGS), in particular DL-α-tocopherol polyethylene glycol succinates such as TPGS-750-M, TPGS-1000, TPGS-1500, TPGS-400, TPGS-1100-M, TPGS-2000, TPGS-860-oleate, TPGS-PEG-PPG-PEG-1100 and TPGS-PPG-PEG-70-butyl, and DL-α-tocopherol polypropylene glycol succinates such as TPPG-1000 and TPPG-1000-butyl and derivatives thereof; and polyethylene glycol α-tocopherol diester of sebacic acid (PTS) such as PTS-600; polyethylene glycol alkyl ether surfactants, such as Brij surfactants, in particular Brij 30, Brij 35, Brij 52, Brij 56, Brij 58, Brij 72, Brij 76, Brij 78, Brij 92, Brij 96, Brij 98; cholesteryl PEG succinates such as cholesteryl PEG1000 succinate; β-sitosterol methoxyethyleneglycol succinate (Nok), in particular Nok comprising mPEG such as mPEG550. All the aforementioned embodiments in the section relating to the use of an amphiphilic surfactant as a stabilizer for protein-containing formulations, are equally applicable to the protein-containing formulations comprising an amphiphilic surfactant.

The concentration of the surfactant in the formulation in particular is at least 0.001% (w/v), in particular at least 0.01% (w/v), especially at least 0.05% (w/v) or at least 0.1% (w/v). In specific embodiments, the concentration of the surfactant in the formulation is in the range of 0.001 to 1.0% (w/v), in particular in the range of 0.02 to 0.8% (w/v), in the range of 0.05 to 0.5% (w/v), 0.1 to 0.5% (w/v), such as about 0.01% (w/v), about 0.02% (w/v), about 0.04% (w/v), about 0.05% (w/v), about 0.08% (w/v), about 0.1% (w/v), about 0.2% (w/v), about 0.3% (w/v) or about 0.5% (w/v). In specific embodiments, the concentration of the surfactant in the formulation is about 4 to 20 times, suitably 4 to 10 times, suitably 4 times, 5 times, 6 times, 10 times or 20 times above the critical micelle concentration. In specific embodiments, the concentration of the surfactant in the formulation is least 5 times, 10 times or 20 times above its critical micellar concentration.

In some embodiments, the protein-containing formulations of the present invention can further comprise one or more excipients. Exemplary excipients include, but are not limited to, sugars, (*e.g.,* trehalose, sucrose, maltose, mannitol, sorbitol) a buffer, salt, antioxidant, preservative, solubilizing agent, diluent, excipient, colorant, and/or flavorant. Such sugars include those which are known in the art and which are conventionally used in pharmaceutical formulations. Examples of such buffers, salts, antioxidants, solubilizing agents, preservatives, diluents, excipients, colorants, flavorants are well known in the art.

In certain embodiments, the protein-containing formulation further comprises a buffer. The buffer in particular should be suitable to keep the pH of the formulation at or about a neutral pH. Such pH buffers include many buffers which are known in the art and which are conventionally used in pharmaceutical formulations. In particular, the buffer is selected from the group consisting of TRIS, phosphate, citrate, acetate and ammonia. In case Brij58 or a compound having formula (III) is used as surfactant, succinate and histidine buffers are not preferred. The protein-containing formulation preferably has a pH at which the protein is active and stable. In certain embodiments, the pH value is in the range of from 4.0 to 10.0, in particular from 6.0 to 8.0, especially from 6.5 to 7.5, such as about 7.0.

In certain embodiments, the formulation further comprises an isotonizing agent, for example, sodium, potassium or calcium chloride, preferably in an amount more than 0.1 M.

The viscosity of the liquid formulations of the present invention at room temperature (25 °C) may be 30 mPa•s or less, 20 mPa•s or less, or 15 mPa•s or less.

The formulations of the present invention may further comprise amino acids. Such amino acids include those which are known in the art and which are conventionally used in pharmaceutical formulations see, for example, Remington The Science and Practice of Pharmacy, 22nd Ed. Pharmaceutical Press, 2013, pp. 1049-1070.

The formulations of the present invention may be administered parenterally, for example via injection. Injection includes, for example, systemic and local administrations by subcutaneous injection, intravenous injection, intramuscular injection, and the like. Thus, the formulations of the present invention are particularly suitable for subcutaneous and intravenous administration.

The formulations of the present invention can also be a stable aqueous solution, in particular, a stable aqueous solution ready for use.

A further aspect of the invention relates to kits for use in the methods of the invention. The kit can comprise an amphiphilic surfactant, *e.g.,* an amphiphilic anti-oxidant surfactant, in a form suitable for stabilizing proteins. In certain embodiments, the amphiphilic surfactant is a surfactant according to the present invention having a formula (I), (II) or (III). The kit can further comprise other components, such as therapeutic agents, carriers, buffers, containers, devices for administration/contacting, compositions for transformation, and the like. The kit can be designed for therapeutic use, diagnostic use, and/or research use and the additional components can be those suitable for the intended use. The kit can further comprise labels and/or instructions, *e.g.,* for stabilizing a protein and/or for patient administration. Such labeling and/or instructions can include, for example, information concerning the amount, frequency and method of administration of the formulation comprising the surfactant.

### Methods of protein stabilization

Further provided are methods of stabilizing proteins. In a particular embodiment, a method of stabilizing a protein is provided, comprising, contacting the protein of interest with an amphiphilic surfactant, to produce a liquid formulation comprising the protein and the amphiphilic surfactant, thereby stabilizing the protein. In some embodiments, the method further comprises at least partially drying the liquid composition that comprises the protein and amphiphilic surfactant. Drying of the liquid formulation may commence any time following the contacting of the protein with the amphiphilic surfactant. Any method of drying a liquid formulation may be used, including, but not limited to, freeze-drying, air-drying, spray-drying, spray-freeze-drying, vacuum drying, and or foam drying.

All the aforementioned embodiments in the section describing the use of an amphiphilic surfactant as a stabilizer for protein-containing formulations, which relate to the protein and the amphiphilic surfactant, are equally applicable to the aforementioned methods of protein stabilization.

### Methods of making

The surfactants of the disclosure can be prepared in a number of ways well known to those skilled in the art of organic synthesis. By way of example, surfactants of the present disclosure can be synthesized using the methods described below, together with methods known in the art of synthetic organic chemistry, or variations thereon as appreciated by those skilled in the art.

Generally, the Vitamin E surfactants, *e.g.,* of formula (I) can be prepared according to the Schemes provided *infra.*

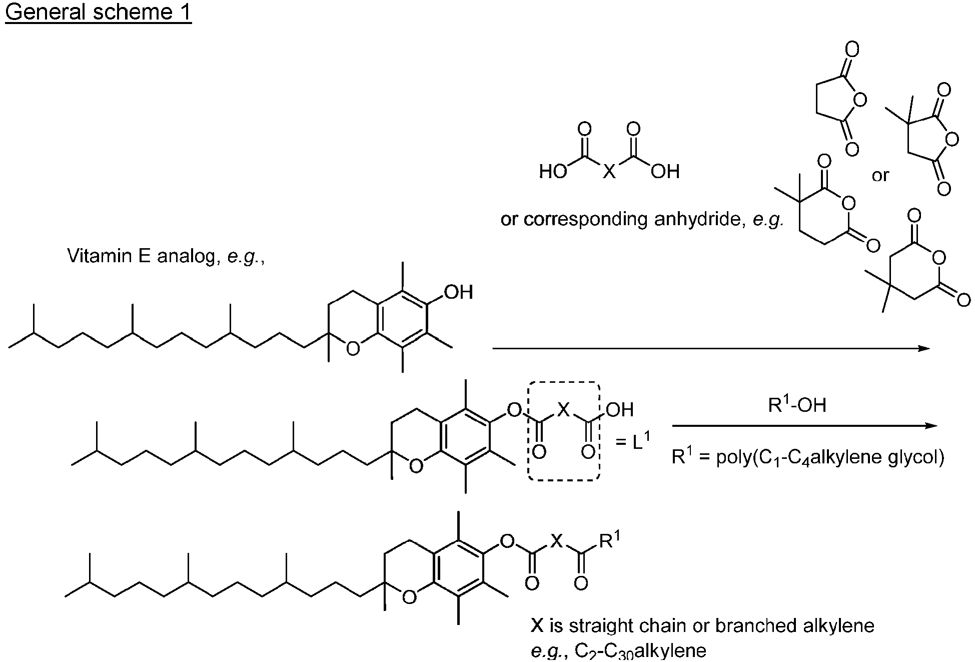

The steps described in General Scheme 1 comprise standard esterification chemistry, the conditions of which are known to the person skilled in the art. 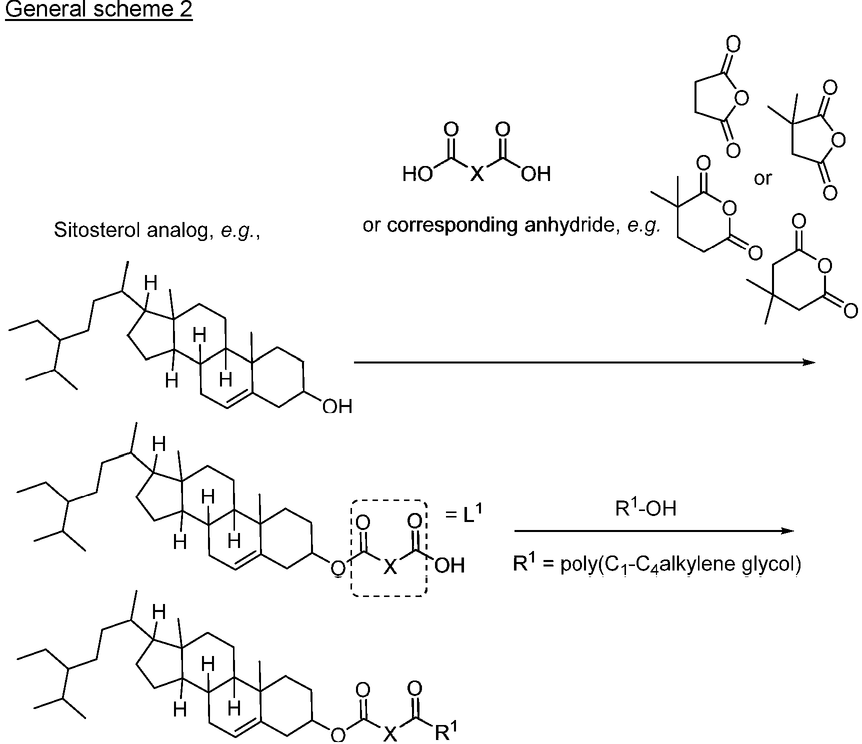
X is straight chain or branched alkylene
*e.g.,* C₂-C₃₀alkylene

The steps described in General Scheme 2 comprise standard esterification chemistry, the conditions of which are known to the person skilled in the art.

The formulations of the present invention can be prepared by mixing the protein with an amphiphilic surfactant, for example, an amphiphilic anti-oxidant surfactant, comprising the step of contacting the protein with an amphiphilic surfactant, to produce a formulation comprising the protein and the amphiphilic surfactant. The formulations produced are in the liquid state.

The formulations of the present invention can be prepared by mixing the protein with an amphiphilic surfactant, *e.g.,* 5% (w/v) surfactant stock solution, which is prepared and sterile filtered using a 0.22 µm filter unit. The necessary stock solution to reach final surfactant concentration is added into the respective protein drug substance.

### Therapeutic uses of the protein-contain formulations

The stable protein-containing formulations of the invention may be useful in therapy. Thus, present invention may be used in pharmaceutical preparations suitable for patient administration. In view of this, the present invention in a further aspect provides a protein-containing formulation for use in therapy, wherein the formulation comprises an amphiphilic surfactant, and wherein the amphiphilic surfactant is present in an amount sufficient to improve the stability of the protein against aggregation. The present invention therefore provides a stable protein-containing formulation for use in therapy, wherein the protein-containing formulation comprises an amphiphilic surfactant.

The invention further provides a method of treating or preventing a disease or disorder in a subject in need thereof, the method comprising administering to the subject a therapeutically effective amount of a protein-containing formulation, wherein the protein-containing formulation comprises an amphiphilic anti-oxidant surfactant, wherein the amphiphilic anti-oxidant surfactant is present in an amount sufficient to improve the stability of the protein against aggregation.

The present invention therefore provides a method of treating or preventing a disease or disorder in a subject in need thereof, the method comprising administering to the subject a therapeutically effective amount of a stable protein-containing formulation comprising an amphiphilic surfactant.

The present invention in a further aspect provides the use of a protein-containing formulation in therapy, wherein the formulation comprises an amphiphilic surfactant, and wherein the amphiphilic surfactant is present in an amount sufficient to improve the stability of the protein against aggregation. The present invention therefore provides the use of a stable protein-containing formulation in therapy, wherein the protein-containing formulation comprises an amphiphilic surfactant.

The present invention in a further aspect provides the use of a protein-containing formulation in the manufacture of a medicament for treating a disease or disorder, wherein the formulation comprises an amphiphilic surfactant, and wherein the amphiphilic surfactant is present in an amount sufficient to improve the stability of the protein against aggregation. The present invention therefore provides the use of a stable protein-containing formulation in the manufacture of a medicament for treating a disease or disorder, wherein the protein-containing formulation comprises an amphiphilic surfactant.

All the aforementioned embodiments in the section relating to protein-containing formulations comprising an amphiphilic surfactant, are equally applicable to the aforementioned formulations for use and methods of treatment.

Other features of the invention will become apparent in the course of the following descriptions of exemplary embodiments which are given for illustration of the invention and are not intended to be limiting thereof.

### Examples

### Synthesis of Surfactants having Formula (I)

### Prototypical procedure:

To a 250 mL round bottom flask with stir bar was added the lipophilic alcohol (1 eq) and the anhydride.(1 eq). Next was added DCM (100 mL), and the flask stirred briefly. Next was added Et₃N (3 eq) and finally DMAP (0.15 eq). At completion, the reaction mixture was transferred to a 500 mL separating funnel and the flask rinsed with 50 mL DCM. The organic layer was washed 3 x 100 1M HCl solution, then washed with 50 mL of 10% brine. The organic phase was dried over anhydrous MgSO₄, filtered, and the organic solvent removed via rotary evaporation. The product was recrystallized from ~200 mL of hot heptanes, cooled to room temperature and filtered. The filter cake was washed with heptanes and the product dried in the vacuum oven.

To a 500 mL round bottom flask with stir bar was added the carboxylic acid (1 eq) generated above, then toluene (200 mL), then PEG1000 (3 eq for the PEG, 1 eq for the PEG-OMe). The flask was stirred briefly to mix, then p-TSA*H₂O (0.15 eq) was added. The flask was placed in an oil bath heated to 140 °C and the flask was fitted with a Dean-Stark trap and condenser, and refluxed. At completion, the reaction was removed from heat, and the solvent removed via rotary evaporation. The crude product was taken up in deionized H₂O (350 mL) and the volatile organic solvent was removed by distillation. The aqueous phase was then extracted with DCM (300 mL) (with gentle agitation to prevent emulsion), resulting into the crude product. The crude product was purified via column chromatography using silica, and liquid loading technique (crude taken up in EtOAc for liquid loading). The column was run with first a 50:50 (EtOAc/hexanes) mixture, then DCM, then a gradient of 0-4% MeOH/DCM.

### Vitamin E analogs

### Example 9: (Reference)

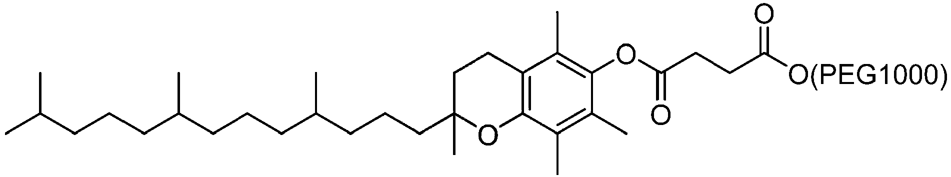

### Example 10:

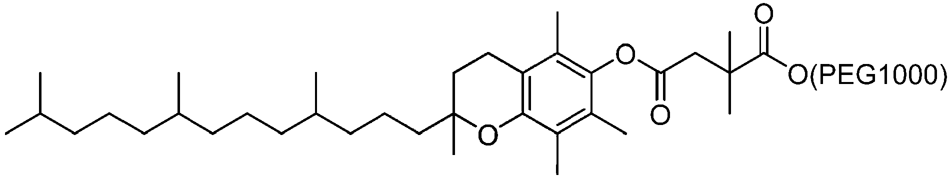

1H NMR (400 MHz, Chloroform-d) δ 4.26 - 4.21 (m, 2H), 3.83 - 3.44 (m, 88H), 2.92 (s, 2H), 2.83 (d, *J =* 5.8 Hz, 1H), 2.57 (t, *J* = 6.8 Hz, 2H), 2.07 (s, 3H), 2.00 (s, 3H), 1.96 (s, 3H), 1.77 (ddq, *J =* 20.0, 13.3, 6.9 Hz, 2H), 1.60-1.46 (m, 4H), 1.37 (s, 10H), 1.32 - 1.17 (m, 12H), 1.17 - 1.00 (m, 7H), 0.90 - 0.79 (m, 13H).

13C NMR (101 MHz, CDCl3) δ 176.39, 169.88, 149.45, 126.59, 124.84, 122.91, 117.28, 74.98, 72.55, 70.55, 70.33, 69.06, 69.02, 63.78, 61.66, 43.73, 40.50, 39.33, 37.52, 37.49, 37.43, 37.40, 37.36, 37.34, 37.31, 37.29, 37.24, 32.73, 32.71, 32.66, 32.64, 31.07, 31.05, 27.92, 25.68, 25.43, 24.76, 24.74, 24.38, 23.87, 22.69, 22.60, 20.98, 20.57, 19.73, 19.67, 19.64, 19.61, 19.58, 13.00, 12.15, 11.77.

### Example 11:

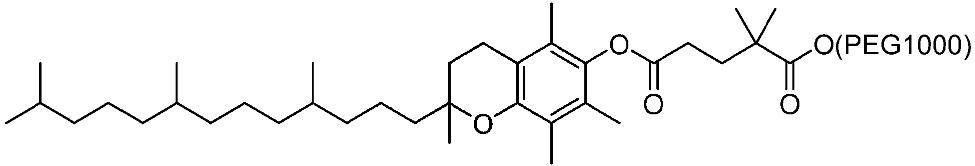

1H NMR (400 MHz, Chloroform-*d*) δ 4.30 - 4.23 (m, 2H), 3.86 - 3.42 (m, 88H), 2.79 (t, *J* = 6.1 Hz, 1H), 2.59 (ddd, *J* = 8.0, 6.1, 3.2 Hz, 4H), 2.08 (s, 3H), 2.00 (s, 3H), 1.96 (s, 3H), 1.78 (ddt, *J* = 20.6, 13.5, 6.9 Hz, 2H), 1.52 (dt, *J* = 13.2, 6.6 Hz, 4H), 1.39 (qt, *J* = 9.2, 4.6 Hz, 4H), 1.25 (d, *J* = 13.2 Hz, 16H), 1.17 - 0.99 (m, 7H), 0.90 - 0.79 (m, 13H).

13C NMR (101 MHz, CDCl3) δ 176.95, 171.88, 149.29, 140.36, 126.54, 124.79, 122.90, 117.27, 74.96, 72.50, 70.56, 70.52, 70.39, 70.30, 69.07, 63.55, 61.62, 41.74, 39.30, 37.47, 37.45, 37.37, 37.33, 37.31, 37.28, 37.25, 37.21, 35.18, 32.70, 32.68, 32.62, 32.60, 29.89, 27.90, 25.00, 24.74, 24.72, 24.36, 22.69, 22.59, 20.96, 20.54, 19.72, 19.65, 19.62, 19.59, 19.56, 12.94, 12.09, 11.78.

### Example 12:

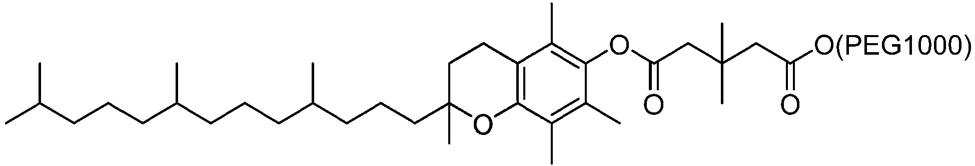

1H-NMR (DMSO-d₆, 400 MHz): δ_{H} 4.55 (m, 0.5H), 4.15-4.13 (m, 2H), 3.61-3.40 (m, 80H), 2.76 (s, 2H), 2.55-2.51 (m, 3H), 2.00 (s, 3H), 1.92-1.89 (d, J = 8.4 Hz, 6H), 1.75-1.74 (m, 2H), 1.51-1.04 (m, 31H), 0.84-0.80 (m, 12H) ppm

### Example 13: (Reference)

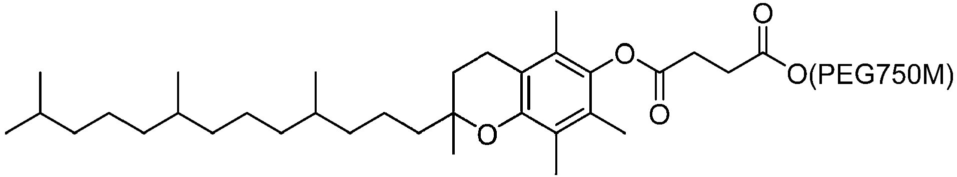

### Example 14:

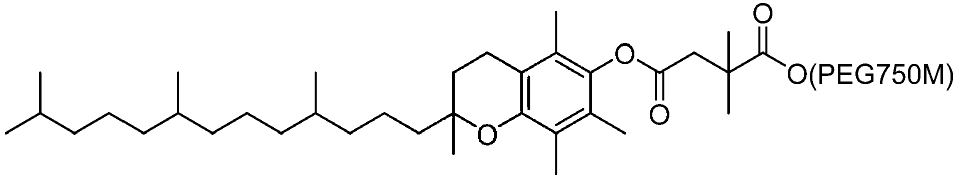

1H NMR (DMSO-d6, 400 MHz): δ 4.12 (m, 2H), 3.58-3.56 (m, 2H), 3.51-3.49 (m, 57H), 3.44-3.41 (m, 2H), 3.24 (m, 3H), 2.74 (s, 1H), 2.53 (m, 2H), 2.00 (m, 3H), 1.91-1.88 (m, 6H), 1.48-1.42 (m, 2H), 1.47- 1.42 (m, l0H), 1.35-1.03 (m, 20H), 0.83-0.79 (m, 12H) ppm.

### Example 15:

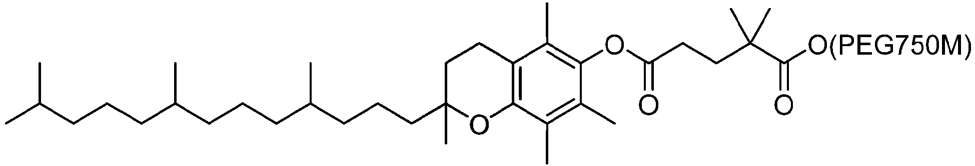

1H-NMR (DMSO-d6, 400 MHz): δ 4.16-4.13 (m, 2H), 3.58-3.50 (m, 2H), 3.49-3.43 (m, 53H), 3.42- 3.41(111, 2H), 3.24 (s, 3H), 2.75 (s, 2H), 2.54-2.51 (m, 2H), 2.50-2.49 (m, 2H), 2.00 (s, 3H), 1.90 (d, J = 9.2 Hz, 6H), 1.79-1.65 (m, 2H), 1.50-1.47 (m, 3H), 1.36-1.01 (m, 28H), 0.88-0.79 (m, 12H) ppm

### Example 16:

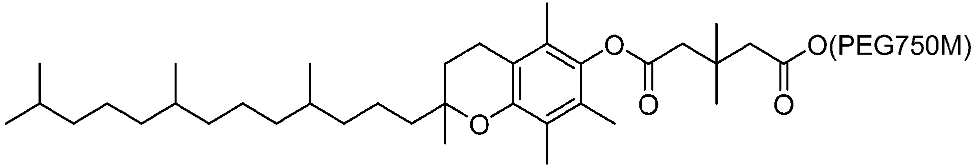

### Data for Surfactant Formulations

### Example 18: Preparation of Formulations According to the Invention

All surfactants were solubilized in water for injection (WFI) generating a 5% (w/v) stock solution. The solution was sterile filtered through a 0.22 µm filter unit. Stock solution was stored at -20 °C till use.

In this section the following proteins were used to demonstrate the broad applicability of the invention across several protein classes. Protein 1 is a disordered protein. Protein 2 is a fab, and Protein 3 is a monoclonal antibody.

### 18.1: Preparation of a formulation comprising disordered protein: Protein 1

The API corresponding buffer was prepared with 20 mM Histidine, 220 mM Succrose pH 5.8 in water. The respective surfactant stock solution in question was added into the buffer accordingly in order to reach the final surfactant concentration (Brij58succinate 0.01% (w/v); Vit E variants 0,04% (w/v); SPGS-550-M 0.01% (w/v))

### 18.2: Preparation of a fab-containing formulation: Protein 2

The API corresponding buffer was prepared with 10 mM Histidine pH 5 in water. The respective surfactant stock solution in question was added into the buffer accordingly in order to reach the final surfactant concentration (Brij58succinate 0.01% (w/v); VitE variants 0.04% (w/v); SPGS-550-M 0.01% (w/v))

### 18.3: Preparation of a monoclonal antibody formulation: Protein 3

The API corresponding buffer was prepared with 3.05 mg/mL Adipic Acid pH 7 in water. The respective surfactant stock solution in question was added into the buffer accordingly in order to reach the final surfactant concentration (Brij58succinate 0.01% (w/v); VitE variants 0.04% (w/v); SPGS-550-M 0.01% (w/v))

### Example 19: Mechanism of action of amphiphilic surfactants in protein-containing liquid formulations

### 19.1 Aim:

The aim of this experiment was to investigate the type of stabilization mechanism which the surfactants employ in protein-containing formulations. A Pendant-Drop Tensiometer was utilized to determine protein (API) displacement by surfactants at the air/water interface. Surfactant interface saturation mitigates the risk of interfacial stress for proteins and associated negative effects potentially impacting protein quality attributes.

### 19.2 Sample preparation:

The protein corresponding buffer was prepared and sterile filtered using a 0.22 µm filter unit. The surfactant in question was added into the buffer using a 5% stock solution as described earlier. The buffer surfactant mix was aliquoted in 2 Nalgene bottles. To one of the two bottles was added the respective API in question to reach a final concentration of 1 mg/mL for protein 1 and 10 mg/mL for proteins 2 and 3.

### 19.3 Method:

Equilibrium surface tension was measured using the PAT-1M tensiometer (Sinterface). The system was rinsed 12 times with the sample to be measured. A hanging drop of constant volume (20 µL) was measured every second for 60 min. Drug substance samples with and without surfactants were measured at the same day to avoid operator, sample and day to day variations.

### 19.4 Result:

### 19.4.1 Vitamin E derivatives

VEDS= Vitamin E dimethyl succinate (Example 10); VEDG2.2 = Vitamin E dimethyl glutarate (Example 12)

Line (a) of Figures 1A-1C show that protein 1 without surfactant shows to be surface active as the surface tension decreases over time. Formulations with surfactants TPGS1000 in Figure 1A and VEDS and VEDG2.2 in Figures 1B and 1C show an even higher surface activity resulting in significant drop of surface tension. Therefore, surfactant displaces the protein from the interface mitigating interfacial stress.

Figures 1A-1C show displacement of protein 1. Same mechanism holds true for protein 2 (Fab) and protein 3 (mAb). The data suggest that the amphiphilic surfactants, e.g., Vitamin E surfactants operate mainly via the displacement mechanism since the surfactant displaces the protein from the interface due to its higher surface activity resulting in lower surface tension (line (c)).

### 19.4.2 Sitosterol derivatives (Reference)

As shown by Figure 1D, line (a), protein 1 without surfactant shows to be surface active as the surface tension decreases over time. Formulations with the SPGS-550-M (NOK) surfactant in the absence or presence of protein 1 (lines (b) and (c), respectively) show an even higher surface activity resulting in significant drop of surface tension. Therefore, the amphiphilic surfactant, e.g., NOK, displaces the protein from the interface mitigation interfacial stress.

Figure 1D shows displacement of the smallest model protein 1. The same mechanism holds true for protein 2 (Fab) and protein 3 (mAb). The data suggest that the amphiphilic surfactants, e.g., sitosterol derivatives, such as SPGS-550-M operate via the displacement mechanism since the surfactant displaces the protein from the interface due to its higher surface activity resulting in lower surface tension (line (c)). As the surface tension does not drop to a surface tension level of about 40 mN/m it cannot be excluded that this surfactant is also employing the preferential associate mechanism as it is less surface active compared to polysorbate.

### 19.4.3 Poly(alkylene glycol) alkyl ether derivatives (Reference)

Line (a) of Figures 1E and 1F show that protein 1 without surfactant shows to be surface active as the surface tension decreases over time. Formulations with surfactants Brij58 and Brij58succinate in the absence or presence of protein 1 (lines (b) and (c), respectively) show an even higher surface activity resulting in significant drop of surface tension. Therefore, surfactant displaces the protein from the interface mitigation interfacial stress.

Figures 1E and 1F show displacement of the smallest model protein 1. Same mechanism holds true for protein 2 (Fab) and protein 3 (mAb). The data suggest that amphiphilic surfactants, e.g., Brij58 and Brij58succinate operate mainly via the displacement mechanism since the surfactant displaces the protein from the interface due to its higher surface activity resulting in lower surface tension (line (c)).

### Example 20: Assessment of the stabilization effect of amphiphilic surfactants as protein (API) stabilizers in liquid formulations

### 20.1 Aim:

The aim of this experiment was to investigate the effect of amphiphilic surfactants to protect proteins from interfacial stress, such as that which is present during manufacturing, transport and application. As a proxy for this stress the forced degradation assay (orbital shaking), applying mechanical stress, was used in order to determine whether chosen surfactants stabilize three different protein API's (disordered protein, Fab, mAb) against mechanical stress (mitigating particle creation (protein aggregates)).

### 20.2 Sample Preparation:

To each protein API was added the respective amount of surfactant (5% stock solution) needed to reach the final surfactant concentration (Brij58 0.01%; VitE variants 0.04%; SPGS-550-M 0.01%), as described earlier. 1.2 mL of sample was aliquoted in each 6R vial (2x). Vials of same sample were pooled after the study in order to generate enough volume for all analytical testing.

### 20.3 Method:

Mechanical stress was generated through orbital shaking at 250 rpm at ambient temperature for 0 h, 7 h, 24 h and 48 h using an orbital shaker. Samples were subsequently analyzed via micro-flow-imaging (MFI). Particle count ≥2 µm/mL was visualized by MFI.

### 20.4 Result:

### 20.4.1 Pre-screen commercial surfactants compared to Polysorbate

Commercial surfactants Brij58, SPGS-550-M and TPGS-1000 were tested against the current gold standard PS20/80 (Polysorbate 20 and Polysorbate 80) as shown in Figures 2A-2C. The data shown in Figures 2A-2C show that irrespective of the protein class, all amphiphilic surfactants resulted in fewer particles than the current standard (PS20/80). Thus, amphiphilic surfactants which are not derived from fatty acid building blocks, such as Vitamin E, Sitosterol (Reference) and poly(alkylene glycol) alkyl ether derivatives (Reference) have an enhanced stabilization effect in protein containing formulations over polysorbates.

### 20.4.2 Vitamin E derivatives

### VEDS= Vitamin E dimethyl succinate (Example 10); VEDG3.3 = Vitamin E dimethyl glutarate (Example 11); VEDG2.2 = Vitamin E dimethyl glutarate (Example 12)

Figures 2D-2F show that Vitamin E derivatives prevent particle formation . For protein 1, VEDS and VEDG 2.2 perform better than TPGS-1000. Protein 2 as representative of Fab's and protein 3 as representative of mAbs show significantly reduced number of particles counts when the amphiphilic surfactants are present. This demonstrates the API stabilizing potential of the amphiphilic surfactants under mechanical stress.

### 20.4.3 Sitosterol derivatives (Reference)

Figures 2G-2I show that Sitosterol analogs, such as SPGS-550-M (NOK) prevent particle formation for tested protein classes. Protein 2 as representative of Fab's and protein 3 as representative of mAbs show significantly reduced number of particles when surfactants are present. This demonstrates the API stabilizing potential of the amphiphilic surfactants under mechanical stress.

### 20.4.4 Poly(alkylene glycol) alkyl ether derivatives (Reference)

Figures 2J-2L show that poly(alkylene glycol) alkyl ether derivatives, such as Brij58 and Brij58succinate prevent particle formation. For protein 1, Brij58succinate performs better than Brij58. Protein 2 as representative of Fab's and protein 3 as representative of mAbs show a significantly reduced number of particles when the amphiphilic surfactants are present. This demonstrates the API stabilizing potential of the amphiphilic surfactants under mechanical stress.

### Example 21: Assessment of effect of amphiphilic surfactants on stability profile upon chemical degradation (e.g., oxidation, hydrolysis)

### 21.1 Aim:

Polysorbate 20/80 suffers from chemical and enzymatic degradation mechanisms as described earlier. In order to test the amphiphilic surfactants of the disclosure for stability against oxidative and hydrolytic stress, the following forced degradation assay was performed using hydrogen peroxide and various buffers to mimic those stress conditions. The aim was to mimic chemical degradation pathways that surfactants would encounter during shelf life.

### 21.2 Method:

H₂O₂ (0.25 M and 2.5 M) was added to formulations containing surfactants and subsequently incubated for 6 days at 40 °C in order to mimic oxidative stress. In addition, surfactants were added to different buffer types (e.g., histidine, citrate, phosphate and succinate buffer) and exposed to accelerated degradation stress such as temperature stress (50 °C for 1 month) at different pH (5.5 and 6.5) in order to mimic chemical degradation pathways (e.g., oxidation, hydrolysis). Control samples were frozen to avoid degradation (FZ). Chromatography methods were used to determine the surfactant content of formulations after accelerated conditions by comparing it to the amount of surfactant spiked into the buffer at the beginning of the experiment.

### 21.3 Results:

### 21.3.1 Polysorbate80 (Reference)

Figure 3A shows that PS80 is stable upon H₂O₂ treatment for 6 days at 40 °C in 0.25 M and 2.5 M H₂O₂ stress.

The results from Figure 3B show that PS80 is stable in Citrate buffer irrespective of pH or elevated temperatures (e.g., 50 °C for 1 month). PS80 shows decreased content for histidine, phosphate and succinate buffer at accelerated conditions, and is therefore degrading into free fatty acids and peroxides under those conditions.

### 21.3.2 Vitamin E derivatives

As shown in Figure 3C TPGS1000 is stable upon H₂O₂ treatment using 0.25 M and 2.5 M of H₂O₂.

As shown in Figure 3D, TPGS1000 is stable in in all chosen buffer conditions, irrespective of pH variants (pH 5.5 and pH 6.5) and elevated temperatures (e.g., 50°C for 1 month).

### 21.3.3 Poly(alkylene glycol) alkyl ether derivatives (Reference)

As shown in Figure 3E Brij58 is stable upon H₂O₂ treatment. Minor loss of Brij58 occurred in 2.5 M H₂O₂ condition.

Figure 3F shows that Brij58 is stable in citrate and phosphate buffers irrespective of pH or elevated temperatures (e.g., 50 °C for 1 month). Brij58 shows decreased content for histidine and succinate buffer at accelerated conditions.

### 21.4 Summary

The shown amphiphilic surfactants are considered stable in the presence of hydrolytic, oxidative and temperature stress in the chosen buffer conditions (histidine, phosphate, citrate and succinate buffer).

### Example 22: Assessment of safety profile of amphiphilic surfactants in human primary tissue (in vitro)

### 22.1 Aim:

The amphiphilic nature of surfactants poses the risk of surfactant interaction and / or integration into lipid bilayers of mammalian cells. This is a safety concern for patients, for example, when administered to humans. In order to show that the chosen surfactants are safe with regard to hemolysis, dendritic cells activation and basophile cell activation, the following assays were performed (see method section).

### 22.2 Method:

Hemolysis assay: Human whole blood was incubated with surfactants at 37 °C for 1 h. Cells were spun down 10 min at 2000 g. The COBAS6000 biochemistry analyzers take an aliquot of the patient specimen and dilute it in saline solution (0.9 % sodium chloride) to measure the absorbance for hemolysis at 570 nm (primary wavelength) and 600 nm (secondary wavelength). From these absorbance values the instrument calculates serum index values.

Basophile activation assay: Human whole blood was used to purify B-cells using an in vitro B-cell activation kit (Cellonic, Basotest, Cat#10-0500). Basophile cells were incubated in 100 µL 1xwash buffer containing the final surfactant concentration as the correct dilutions were prepared beforehand. Cell labeling, lysis and fixation was performed according to manufacturer instructions and subsequently analyzed using a BD FORTESSA flow cytometer. Leucocytes are gated via forward and sideward scatter. The Basophile subpopulation was gated on CD123 and IgE staining. Finally, activated Basophile cells were identified via CD203c and CD63 gating. All fluorescently labeled antibodies were provided in the kit stated above.

Dendritic cell activation: Human whole blood was used to extract monocytes using MACS (magnetic activated cell sorting) according to manufacturer's instructions (Miltenyi Biotec Order no. 130-114-980). Cells were incubated for 6 days in respective differentiation media (Miltenyi DC differentiation medium order no. 130-094-812) resulting in dendritic cells. Cells were incubated in medium with surfactants in question for 48 h and subsequently stained for cell activation using fluorescently labeled antibodies (Mouse Anti-Human CD14 PerCP-Cy5.5 order no. 562692 from BD to check monocyte negative; Mouse Anti-Human CD11c BV605 from BD to check dendritic cell differentiation and Mouse Anti-Human CD80 FITC order no. 557226 from BD to check cell activation).

### 22.3 Results:

### 22.3.1 Vitamin E derivatives

Hemolysis data as shown in Figure 4A demonstrate no increased hemolytic potential for the Vitamin E derived surfactants compared to current polysorbate standard.

The B-cell activation assay data as shown by Figure 4B showed no elevated B-cell activation of tested surfactants compared to the positive control (activated B-cells). Moreover, VEDS, VEDG 2.2 and VEDG 3.3 perform better than TPGS1000.

The data from the dendritic-cell (DC) activation assay as described in Figure 4C showed no elevated DC levels upon surfactant treatment. Moreover, VEDS, VEDG 2.2 and VEDG 3.3 perform better than TPGS1000.

Thus, the amphiphilic surfactants having formula (I) are envisaged to have no adverse safety effects with regard to hemolytic potential or immune system activation. It is envisaged that surfactants of formula (I) may give an increased safety and/or less toxic safety profile compared to the commercial Vitamin E analogs.

### 22.3.2 Sitosterol derivatives (Reference)

Hemolysis data as shown in Figure 4D demonstrate no increased hemolytic potential for the Sitosterol surfactants compared to current polysorbate standard.

The B-cell activation assay as shown by Figure 4E showed slightly increased levels of B-cell activation which, are still regard as safe.

The data from the dendritic-cell activation assay as described in Figure 4F showed no elevated DC levels upon surfactant treatment.

Thus, the amphiphilic surfactants having formula (II) are envisaged to have no adverse safety effects with regard to hemolytic potential or immune system activation and display a safety profile comparable to polysorbates.

### 22.3.3 Poly(alkylene glycol) alkyl ether derivatives (Reference)

Hemolysis data as shown in Figure 4G demonstrate increased hemolytic potential for Brij58 at a concentration of 0.05% (w/v). Brij58 do not contain a linker between its hydrophobic and hydrophilic portions. Introducing the succinate linker into Brij58 reduced its hemolytic potential to levels comparable of polysorbate20. Hence, surfactant safety for Brij58 increases by introducing a linker derived from a dicarboxylic acid, e.g., succinate linker.

The B-cell activation assay as shown by Figure 4H showed no elevated B-cell activation of tested surfactants compared to positive control (activated B-cells). In accordance with the above result, basophils activation for Brij58 decreases by introducing a linker derived from a dicarboxylic acid, e.g. succinate linker.

The data from the dendritic-cell activation assay as described in Figure 4I showed no elevated DC levels upon surfactant treatment.

Thus, the surfactants with formula (III) are envisaged to have no adverse effects regarding hemolytic potential or immune system activation. It is envisaged that surfactants of formula (III) may give an especially increased safety and/or less toxic safety profile than other amphiphilic surfactants.

### Example 23: Assessment of propensity to inhibition of platelet aggregation of amphiphilic surfactants

### 23.1 Aim:

Surfactants, as intrinsically amphiphilic molecules, display a strong interaction or integration in lipid bilayers. Platelets (thrombocytes) reacts to a blood vessel injury by aggregating into a blood clots. This process is mostly governed by membrane interactions that might be disrupted by the presence of surfactants. The inhibition of this aggregation process, which would cause an impaired hemostasis, can be tested in vitro by activate the platelets (from the whole blood of a healthy donor) with a particular agonist, incubate them with the testing sample and analyzing the degree of platelet inhibition. Therefore, the aim of this experiment was to test the degree of platelet inhibition triggered by the amphiphilic surfactants.

### 23.2 Sample preparation:

Human whole blood of 6 healthy volunteers was incubated with 0.04% or 0.01% surfactants for 10 minutes at 37°C. Saline solution was used as negative control. Platelets were activated with four independent agonists: arachidonic acid (AA), thrombin receptor activator peptide (TRAP6), collagen and ADP. Aggregation was measured through Whole Blood Aggregometry (WBA).

### 23.3 Method:

Whole Blood Aggregometry (WBA) consists of immerging electrodes into whole blood which measure the impedance between them. The addition of a platelet agonist activates the aggregation, resulting in an increase of impedance over time. This increase can be compared between a negative control and a testing sample to derive the degree of platelet inhibition.

### 23.4 Results:

As shown in Figure 5A, the amphiphilic surfactants containing vitamin E, VEDS and VEDG 3.3, display comparable platelet inhibition values as PS80, independently from the agonist employed. As shown in Figure 5B, SPGS-550-M has no effect on platelet aggregation. As shown in Figure 5C, while Brij58 and Brij58-succinate generally show a higher inhibition of the aggregation compared to PS80, the amount of inhibition is still associated with no impaired hemostasis. Moreover, In 3 out of 4 agonists shown in Figure 5C, Brij58-succinate display a lower platelet inhibition compared to Brij58, indicating a the beneficiary effect on the safety profile triggered by the addition of the linker.

### Example 24: Assessment of surfactant's propensity of enzymatic degradation by protein 4 formulation (host cell proteins (HCPs))

### 24.1 Aim:

Polysorbate 20/80 suffer from enzymatic degradation mechanisms as described earlier. In order to test the amphiphilic surfactants of the disclosure for stability against enzymatic degradation, the following assay was performed. The aim was to test whether the amphiphilic surfactants are susceptible to the same enzymatic degradation effective on PS80.

### 24.2 Sample preparation:

Protein 4 is a monoclonal antibody which production process resulted in contaminant HPCs enzymes, a common scenario as previously described. Therefore, a formulation containing protein 4 also contains the enzymes responsible for polysorbate degradations and it is a suitable proxy for testing the propensity of enzymatic degradation during shelf life in real-world conditions. The formulation was complemented with either 0.02% PS80 or 0.04% of the amphiphilic surfactants VEDS, VEDG 2.2 or TPGS-1000 and incubated for one month at either 5°C or 40°C.

### 24.3 Method:

The surfactant content was measured at the required pull point by HPLC and the analysis was performed by a standard baseline correction followed by integration of the main peak, representing the integer (not degraded) surfactant.

### 24.4 Results:

As shown in Figure 6, enzymatic degradation, originating from protein 4 host cell proteins (HCPs), is largely affecting PS80. Degradation is observed both at 5°C and 40°C, with an expected stronger effect at higher temperatures. VEDS, VEDG 2.2 and TPGS-1000 are not affected by enzymatic degradation after one month, irrespective of the incubation temperature.

### Example 25: Assessment of surfactant's propensity for oxidative and hydrolytic degradation in quiescence stability studies

### 25.1 Aim:

Polysorbate 20/80 suffer from chemical degradation mechanisms as described earlier. In order to test the amphiphilic surfactants of the disclosure for stability against oxidative and hydrolytic degradation in long-term studies, a stability study was performed. The aim was to test whether the amphiphilic surfactants are susceptible to chemical degradation affecting PS80 and, if so, to what extent.

### 25.2 Sample preparation:

Protein 2, in the reported formulation, was complemented with either 0.04% PS80, VEDS, VEDG 2.2, TPGS-1000 or 0.01% Brij58 or SPGS-550-M and incubated for six months at 25°C (Figure 7). A pull point was taken every month and a half (T0, T1.5m, T3m, T4.5m and T6m) and frozen for storage.

### 25.3 Method:

The surfactant content was measured for all pull points of a specific surfactant in a single series by HPLC and the analysis was performed by a standard baseline correction followed by integration of the main peak, representing the integer (not degraded) surfactant.

### 25.4 Results:

As shown in Figure 7, chemical degradation is drastically affecting PS80 in this long-term study with only a small amount of surfactant left after six months (~5%). VEDS, TPGS-1000 and Brij58 are clearly not susceptible to the same degradation, showing stable levels of surfactant contents. VEDG 2.2 possesses a minimal susceptibility to chemical degradation, displaying an initial drop followed by a stable level of surfactant contents around 88-90%. SPGS-550-M is susceptible to chemical degradation, exhibiting an almost linear degradation but not as severe as for PS80.

### Example 26: Comparison of a therapeutic protein (TP) stabilization effect provided by currently used surfactants versus VEDS

### 26.1 Aim:

Comparison of a therapeutic protein (TP) stabilization effect provided by currently used surfactants versus VEDS

### 26.2 Sample preparation:

A liquid formulation containing Protein 5 was prepared as herein described. It was complemented with either 0.2% PLX188 or 0.04% PS20, PS80 or VEDS and incubated for one month at 30°C (Figure 8). A pull point was taken after this time and used for the following analysis.

### 26.3 Method:

The purity of the protein was assessed by SEC-UPLC by standard baseline correction followed by integration of the main peak, identified by comparison with a standard sample, while the sum of impurities was calculated by integration of secondary peaks.

### 26.4 Results:

As shown in Figure 8A and Figure 8B, while all surfactants tested displayed satisfactory levels of API stabilization (>90% purity), a relative comparison between different surfactants results in a ranking that might influence the results in a longer study (i.e. 12 months). PS20 possess the lower purity and higher impurities. PLX188, PS80 and VEDS retain a similar level of purity and impurities, highlighting that the amphiphilic surfactant VEDS possess a perfectly viable stabilization effect.

### Summary

The data described herein show that amphiphilic surfactants show less surfactant degradation upon oxidative and hydrolytic stress compared to polysorbate 20/80. Since Vitamin E analogs do not contain fatty acids as building blocks there is less risk of protein degradation resulting in visible or sub visible particles in addition to less peroxide creation upon surfactant degradation. An enhanced stabilization effect has been shown for those amphiphilic surfactants formed containing the the branched dicarboxylic acid alkyl linker.

Finally, in vitro safety data suggest that the amphiphilic surfactants of the present disclosure do not cause negative effects in human primary tissue with regards to hemolysis, B-cell, or dendritic cell activation and are therefore suited as excipients for parenteral formulations.

Therefore, we have demonstrated a new finding of amphiphilic surfactants as stabilizers of protein APIs in liquid formulations and developed novel surfactants of formula (I) with predictable surfactant characteristics, physiologic tolerability and none to minimal drawbacks compared to those known from polysorbates, in particular with respect to excipient instability.

## Claims

1. A protein-containing formulation comprising a buffer and an amphiphilic surfactant, wherein the amphiphilic surfactant is a compound having the formula (I): 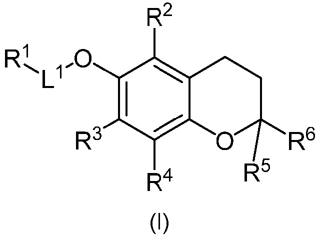 wherein:
for formula (I):
L¹ is -C(=O)-X-C(=O)- ;
X is a branched C₂-C₂₀alkylene;
R¹ is a poly(C₁-C₄-alkylene glycol) group, e.g., PEG group, optionally wherein the poly(C₁-C₄-alkylene glycol) group terminates in hydroxyl or C₁-C₆alkoxyl;
R², R³, R⁴ and R⁵ are each independently selected from hydrogen and C₁-C₁₀alkyl; and
R⁶ is C₃-C₂₀alkyl.

2. The formulation according to claim 1, wherein the amphiphilic surfactant is an amphiphilic antioxidant surfactant.

3. The formulation according to any one of the preceding claims, wherein the protein is a disordered protein or an antibody.

4. The formulation according to claim 3, wherein the antibody is an antibody-drug conjugate, monoclonal antibody or antigen-binding fragment.

5. The formulation according to any one of claims 3 and 4, wherein the antibody is present in an amount of 1-250 mg/ml.

6. The formulation according to any one of claims 1 to 5, wherein the compound having the formula (I) comprises a poly(alkylene glycol) moiety as the hydrophilic part of the surfactant molecule.

7. The formulation according to any one of claims 1 to 6, wherein the amphiphilic surfactant is a compound having the formula (I), wherein
R' is a PEG group, which PEG group terminates in hydroxyl or C₁-C₆alkoxyl.

8. The formulation according to any one of claims 1 to 7, which is for subcutaneous administration.

9. Use of an amphiphilic surfactant as a stabilizer for a formulation comprising a protein, wherein the amphiphilic surfactant is a compound having the formula (I) as defined according to claim 1 or claim 7.

10. The use according to claim 9, wherein the protein is defined according to any one of claims 3 to 5.

11. A surfactant of formula (I): 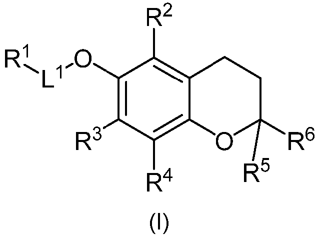 wherein for formula (I):
L¹ is -C(=O)-X-C(=O)- ;
X is a branched C₂-C₂₀alkylene;
R¹ is a poly(C₁-C₄-alkylene glycol) group, e.g., PEG group;
R², R³, R⁴ and R⁵ are each independently selected from hydrogen and C₁-C₁₀alkyl; and
R⁶ is C₅-C₂₀alkyl.

12. The surfactant according to claim 11, wherein the amphiphilic surfactant is a compound having the formula (I), wherein
R' is a PEG group, which PEG group terminates in hydroxyl or C₁-C₆alkoxyl.

13. A method for *in vitro* preventing or suppressing protein aggregation formation of a protein-containing formulation by using an amphiphilic surfactant as a stabilizer in the formulation, wherein the amphiphilic surfactant is defined according to any one of claims 11 to 12.

14. The method of claim 13, wherein the protein is defined according to any one of claims 3 to 5.

## Patentansprüche

1. Proteinhaltige Formulierung, umfassend einen Puffer und ein amphiphiles Tensid, wobei es sich bei dem amphiphilen Tensid um eine Verbindung mit der Formel (I) handelt: 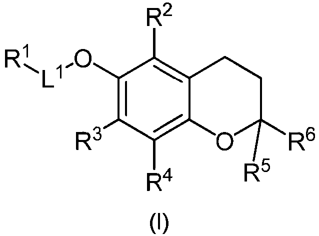 wobei:
bei Formel (I):
L¹ für -C(=O)-X-C(=O)- steht;
X für ein verzweigtes C₂-C₂₀-Alkylen steht;
R¹ für eine Poly(C₁-C₄-alkylenglykol)-Gruppe, z. B. PEG-Gruppe, steht, wobei die Poly(C₁-C₄-alkylenglykol)-Gruppe gegebenenfalls in Hydroxyl oder C₁-C₆-Alkoxyl endet;
R², R³, R⁴ und R⁵ jeweils unabhängig aus Wasserstoff und C₁-C₁₀-Alkyl ausgewählt sind und
R⁶ für C₅-C₂₀-Alkyl steht.

2. Formulierung nach Anspruch 1, wobei es sich bei dem amphiphilen Tensid um ein amphiphiles antioxidatives Tensid handelt.

3. Formulierung nach einem der vorhergehenden Ansprüche, wobei es sich bei dem Protein um ein ungeordnetes Protein oder einen Antikörper handelt.

4. Formulierung nach Anspruch 3, wobei es sich bei dem Antikörper um ein Antikörper-Arzneimittel-Konjugat, einen monoklonalen Antikörper oder ein antigenbindendes Fragment handelt.

5. Formulierung nach einem der Ansprüche 3 und 4, wobei der Antikörper in einer Menge von 1-250 mg/ml vorliegt.

6. Formulierung nach einem der Ansprüche 1 bis 5, wobei die Verbindung mit der Formel (I) eine Poly(alkylenglykol)-Gruppierung als hydrophilen Teil des Tensidmoleküls umfasst.

7. Formulierung nach einem der Ansprüche 1 bis 6, wobei es sich bei dem amphiphilen Tensid um eine Verbindung mit der Formel (I) handelt, wobei
R¹ für eine PEG-Gruppe steht, die in Hydroxyl oder C₁-C₆-Alkoxyl endet.

8. Formulierung nach einem der Ansprüche 1 bis 7, das der subkutanen Verabreichung dient.

9. Verwendung eines amphiphilen Tensids als Stabilisator für eine Formulierung, die ein Protein umfasst, wobei es sich bei dem amphiphilen Tensid um eine wie nach Anspruch 1 oder Anspruch 7 definierte Verbindung mit der Formel (I) handelt.

10. Verwendung nach Anspruch 9, wobei das Protein nach einem der Ansprüche 3 bis 5 definiert ist.

11. Tensid der Formel (I): 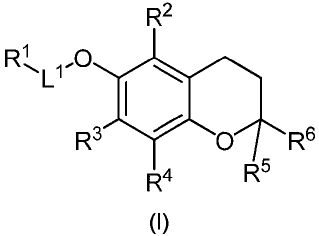 wobei bei Formel (I):
L¹ für -C(=O)-X-C(=O)- steht;
X für ein verzweigtes C₂-C₂₀-Alkylen steht;
R¹ für eine Poly(C₁-C₄-alkylenglykol)-Gruppe, z. B. PEG-Gruppe, steht;
R², R³, R⁴ und R⁵ jeweils unabhängig aus Wasserstoff und C₁-C₁₀-Alkyl ausgewählt sind und
R⁶ für C₅-C₂₀-Alkyl steht.

12. Tensid nach Anspruch 11, wobei es sich bei dem amphiphilen Tensid um eine Verbindung mit der Formel (I) handelt, wobei
R¹ für eine PEG-Gruppe steht, die in Hydroxyl oder C₁-C₆-Alkoxyl endet.

13. Verfahren zur In-vitro-Vorbeugung oder Unterdrückung der Proteinaggregationsbildung einer proteinhaltigen Formulierung durch Verwendung eines amphiphilen Tensids als Stabilisator in der Formulierung, wobei das amphiphile Tensid nach einem der Ansprüche 11 bis 12 definiert ist.

14. Verfahren nach Anspruch 13, wobei das Protein nach einem der Ansprüche 3 bis 5 definiert ist.

## Revendications

1. Formulation contenant une protéine comprenant un tampon et un tensioactif amphiphile, le tensioactif amphiphile étant un composé ayant la formule (I) : 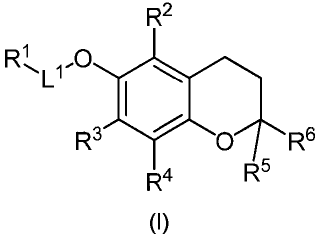 dans laquelle
pour la formule (I) :
L¹ est -C(=O)-X-C(=O)-;
X est un C₂-C₂₀alkylène ramifié ;
R¹ est un groupe poly(alkylène glycol en C₁-C₄), par exemple, un groupe PEG, facultativement dans laquelle le groupe poly(alkylène glycol en C₁-C₄) se termine par hydroxyle ou alcoxyle en C₁-C₆ ;
R², R³, R⁴ et R⁵ sont chacun indépendamment choisis parmi hydrogène et C₁-C₁₀alkyle ; et
R⁶ est C₅-C₂₀alkyle.

2. Formulation selon la revendication 1, dans laquelle le tensioactif amphiphile est un tensioactif antioxydant amphiphile.

3. Formulation selon l'une quelconque des revendications précédentes, dans laquelle la protéine est une protéine désordonnée ou un anticorps.

4. Formulation selon la revendication 3, dans laquelle l'anticorps est un conjugué anticorps-médicament, un anticorps monoclonal ou un fragment de liaison d'antigène.

5. Formulation selon l'une quelconque des revendications 3 et 4, dans laquelle l'anticorps est présent en une quantité de 1-250 mg/ml.

6. Formulation selon l'une quelconque des revendications 1 à 5, dans laquelle le composé ayant la formule (I) comprend un groupement poly(alkylène glycol) en tant que partie hydrophile de la molécule de tensioactif.

7. Formulation selon l'une quelconque des revendications 1 à 6, le tensioactif amphiphile étant un composé ayant la formule (I), dans laquelle
R¹ est un groupe PEG, lequel groupe PEG se termine par hydroxyle ou alcoxyle en C₁-C₆.

8. Formulation selon l'une quelconque des revendications 1 à 7, qui est destinée à une administration sous-cutanée.

9. Utilisation d'un tensioactif amphiphile en tant que stabilisant pour une formulation comprenant une protéine, dans laquelle le tensioactif amphiphile est un composé de formule (I) telle que définie selon la revendication 1 ou la revendication 7.

10. Utilisation selon la revendication 9, dans laquelle la protéine est définie selon l'une quelconque des revendications 3 à 5.

11. Tensioactif de formule (I) : 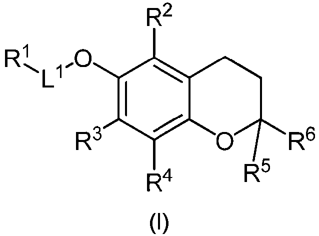 dans laquelle pour la formule (I) :
L¹ est -C(=O)-X-C(=O)-;
X est un C₂-C₂₀alkylène ramifié ;
R¹ est un groupe poly(alkylène glycol en C₁-C₄ ), par exemple un groupe PEG ;
R², R³, R⁴ et R⁵ sont chacun indépendamment choisis parmi hydrogène et C₁-C₁₀alkyle ; et
R⁶ est C₅-C₂₀alkyle.

12. Tensioactif selon la revendication 11, dans lequel le tensioactif amphiphile est un composé ayant la formule (I), dans laquelle
R¹ est un groupe PEG, lequel groupe PEG se termine par hydroxyle ou alcoxyle en C₁-C₆.

13. Procédé pour prévenir ou supprimer *in vitro* la formation d'agrégation protéique d'une formulation contenant des protéines en utilisant un tensioactif amphiphile en tant que stabilisant dans la formulation, dans lequel le tensioactif amphiphile est défini selon l'une quelconque des revendications 11 à 12.

14. Procédé selon la revendication 13, dans lequel la protéine est définie selon l'une quelconque des revendications 3 à 5.
